# EUROPEAN PATENT SPECIFICATION

(11) **EP 3 194 614 B1**
(45) Date of publication and mention of the grant of the patent: **02.05.2018**
(21) Application number: 15751146.0
(22) Date of filing: 27.07.2015
(51) Int. Cl.: C12Q 1/68

(54) **NOVEL TEST FOR MICROBIAL BLOOD INFECTIONS**
NEUARTIGER TEST FÜR MIKROBIELLE BLUTINFEKTIONEN
NOUVEAU TEST POUR INFECTIONS DU SANG MICROBIENNES

(30) Priority: 25.07.2014 NL 2013266
(43) Date of publication of application: 26.07.2017
(73) Proprietor: Microbiome Ltd., 1081 HZ Amsterdam (NL)
(72) Inventor: SAVELKOUL, Paul, Hendrik, Maria, 1081 HZ Amsterdam (NL)
(74) Representative: V.O.
(86) International application number: PCT/NL2015/050548
(87) International publication number: WO 2016/013940

(56) References cited:
- WO-A1-2011/104025
- US-A1- 2008 138 808
- CULBERTSON JUSTIN E ET AL: "Expression and characterization of PhzE from P. aeruginosa PAO1: aminodeoxyisochorismate synthase involved in pyocyanin and phenazine-1-carboxylate production", January 2013 (2013-01), BIOCHIMICA ET BIOPHYSICA ACTA, VOL. 1834, NR. 1, PAGE(S) 240-246, XP002733903, ISSN: 1570-9639(print) page 241, left-hand column, paragraph 2

## Description

### 1. FIELD OF THE INVENTION

The present invention relates to methods and compositions for diagnosing or predicting for microbial blood infections and/or stages of progression in a subject. The invention also relates to methods and compositions for treatment of microbial blood infections and to methods and compositions for monitoring the efficacy of anti-sepsis treatment.

### 2. BACKGROUND OF THE INVENTION

Microbial blood infections such as sepsis are a major and increasing cause of in-hospital morbidity and mortality. In terms of (additional) hospitalisations, it accounts for patient numbers comparable to those for breast and lung cancer. The mortality rate associated with septic shock (i.e. patients with sepsis complicated by strongly reduced blood pressure) is as high as 45%. There is therefore an urgent need to improve diagnosis and therapy planning for microbial blood infections such as sepsis.

Patients are diagnosed as suffering from sepsis when they develop clinical signs of infections or systemic inflammation. A list of signs and symptoms are used in order to make a diagnosis of sepsis, including abnormalities of body temperature, heart rate, respiratory rate, and white blood cell count.

There are different types of sepsis characterized by the type of infecting organism. Sepsis caused by gram-negative pathogens is considered as the most frequent, whereby the majority of gram-negative sepsis is caused by *Escherichia coli, Klebsiella pneumoniae* and *Pseudomonas aeruginosa.* Gram-positive pathogens such as *Staphylococci* and *Streptococci* are the second major cause of sepsis, but their incidence is rising in the last decade.

A third major group includes fungi, with fungal infections causing a relatively small percentage of sepsis cases, but with a high mortality rate.

Blood culture is currently the gold standard for diagnosis but its diagnostic impact is negatively affected by considerable turnaround time and a suboptimal sensitivity (Squire et al. 1979. Pediatrics 64: 60-4; Pierce et al. 1984. Pediatr Infect Dis 3: 510-3).

A confirmed diagnosis as to the type of infection traditionally requires microbiological analysis involving inoculation of blood cultures, incubation for 18- 24 hours, plating the causative microorganism on solid media, another incubation period, and final identification 1-2 days later. Even with immediate and aggressive treatment, some patients can develop multiple organ dysfunction syndrome and eventually die before treatment becomes effective. Since half of all deaths occur within the first three days after blood cultures are obtained, faster and more sensitive identification of the causative pathogen could be of great value to allow an early diagnosis and faster guidance of therapy (Stoll et al. 2002. Pediatrics 110: 285-91).

In addition, the sensitivity is mainly affected by the small volume of blood inoculated in blood cultures, previous administration of antibiotics and presence of low or intermittent bacteremia (Schelonka et al. 1996. J Pediatr 129: 275-8.; Connell et al. 2007. Pediatrics 119: 891-6). The effect of blood volume was clearly demonstrated in a study which showed that blood cultures containing an adequate blood volume were twice more likely to yield a positive result compared to blood cultures with an inadequate blood volume (Connell et al. 2007).

Hence, there remains a strong need for improved techniques for diagnosis and treatment of patients with infectious diseases, blood-borne infections, sepsis, or systemic inflammatory response syndrome. The ability to rapidly detect infectious pathogens would have great value for preventing infections and especially sepsis in the population.

### 3. SUMMARY OF THE INVENTION

The present invention provides methods and kits for diagnosing microbial blood infections such as sepsis in mammals, preferably humans, including neonatals and adults. These methods and kits provide rapid and accurate information about the organism that is responsible for the infection. These near patient tools are easy to use, at or close to the patient's bedside, and will provide rapid information to the physician about how to treat each individual patient in the best way.

The invention therefore provides a method for detecting microbial blood infections as described in claim 1. In a preferred method of the invention, the nucleic acid amplification for the indicated gene or gene product, or a part thereof, is a PCR reaction using a forward primer and a reverse primer comprising a primer sequence as listed beneath, or a part thereof.

In a preferred method of the invention, the nucleic acid amplification for the indicated gene or gene product, or a part thereof, is a PCR reaction using parts of a forward primer and a reverse primer sequence as listed beneath.

In a preferred method of the invention, the nucleic acid amplification for the indicated gene or gene product, or a part thereof, is a PCR reaction using a probe comprising the probe sequence as listed beneath, the complement of the probe sequence as listed beneath; or a part thereof.

In a preferred method of the invention, the nucleic acid amplification for the indicated gene or gene product, or a part thereof, is a PCR reaction using a probe comprising parts of a probe sequence as listed beneath, the complement of the probe sequence as listed beneath; or a part thereof.

In a preferred method of the invention, the nucleic acid amplification reaction of the *phzE* gene or *phzE* gene product, or a part thereof, is a PCR reaction using as a forward primer the sequence 5'-GCCGAGGTCATGGAATTC-3', using as a reverse primer the sequence 5'-ATCCGCGCCATCATCTTC-3', and using as a probe the sequence 5'-CGACAACCGCAAGGAAGCCGA-3'; the nucleic acid amplification reaction of the *rhaA* gene or *rhaA* gene product, or a part thereof, is a PCR reaction using as a forward primer the sequence 5'-AACCAGGCGTCGATAAT-3', using as a reverse primer the sequence 5'-GTTTACGGCGCAATCC-3', and using as a probe the sequence 5'-ACAGGAAAGACAAGACTATGCAGACC-3; and the nucleic acid amplification reaction of the *tuf* gene or *tuf* gene product, or a part thereof, is a PCR reaction using as a forward primer the sequences 5'-CCAACTCCAGAACGTGATTCTG-3', 5'-CCAACTCCAGAACGTGACTCTG-3' and 5'-CCAACACCAGAACGTGATTCTG-3', using as reverse primers the sequences 5'-GTTGTCACCAGCTTCAGCGTAGT-3', 5'-GTTATCACCAGCTTCAGCGTAAT-3', and 5'-GTTGTCACCAGCTTCAGCATAGT-3', and using as a probe the sequence 5'-ACAGGCCGTGTTGAACGTGGKCAAATCAA-3'.

The invention further provides a method for detecting microbial blood infections comprising the steps of: a) performing on a blood sample from a subject suspected of suffering from microbial blood infections, or on a sample of nucleic acids isolated therefrom, a nucleic acid amplification reaction, b) determining the presence and/or amount of amplified nucleic acid produced by said nucleic acid amplification reaction, and c) determining that said subject is suffering from microbial blood infections when said amount of amplified nucleic acid is higher than that produced in a control sample, wherein said nucleic acid amplification reaction comprises the amplification of the *phzE* gene or *phzE* gene product, or a part thereof, of *Pseudomonas aeruginosa,* preferably wherein said nucleic acid amplification reaction is a PCR reaction using as a forward primer the sequence 5'-GCCGAGGTCATGGAATTC-3', using as a reverse primer the sequence 5'-ATCCGCGCCATCATCTTC-3', and using as a probe the sequence 5'-CGACAACCGCAAGGAAGCCGA-3'.

A nucleic acid amplification reaction comprising the amplification of the *phzE* gene or *phzE* gene product is new and was shown to provide adequate performance *in silico* as well as *in vitro,* meaning high sensitivity with a lower detection limit of about 1-5 colony forming unit-equivalents of *P*. *aeruginosa* microorganisms per real time PCR reaction, and high specificity. No positive PCR results were obtained when this test was performed on DNA isolates from 44 different, non-*P*. *aeruginosa* cultures. The sensitive nucleic acid amplification provides a detection sensitivity of about 5-7 micro-organisms per ml of blood. This approaches the sensitivity of blood culturing, the current gold standard for sepsis diagnosis. Moreover, the results from this method for detecting sepsis can be available within 4 hours, while the classical blood cultures take 2-3 days. This fast detection enables fast, adequate antibiotic treatment which will significantly reduce mortality (Kumar et al., 2006. Crit Care Med 34: 1589-96).

The invention further provides a method for detecting microbial blood infections comprising the steps of: a) performing on a blood sample from a subject suspected of suffering from microbial blood infections, or on a sample of nucleic acids isolated therefrom, a nucleic acid amplification reaction, b) determining the presence and/or amount of amplified nucleic acid produced by said nucleic acid amplification reaction, and c) determining that said subject is suffering from microbial blood infections when said amount of amplified nucleic acid is higher than that produced in a control sample, wherein said nucleic acid amplification reaction comprises the amplification of the *rhaA* gene or *rhaA* gene product, or a part thereof, of *Klebsiella* spp.. This test shows high sensitivity, with a lower detection limit of about 1 colony forming unit-equivalents of *Klebsiella* spp. microorganisms per real time PCR reaction, and high specificity. Preferably said nucleic acid amplification of the *rhaA* gene or *rhaA* gene product, or a part thereof, of *Klebsiella* spp reaction is a real-time PCR reaction using as a forward primer the sequence 5'-AACCAGGCGTCGATAAT-3', using as a reverse primer the sequence 5'-GTTTACGGCGCAATCC-3', and using as a probe the sequence 5'-ACAGGAAAGACAAGACTATGCAGACC-3'.

The invention further provides a method for detecting microbial blood infections comprising the steps of: a) performing on a blood sample from a subject suspected of suffering from microbial blood infections, or on a sample of nucleic acids isolated therefrom, a nucleic acid amplification reaction, b) determining the presence and/or amount of amplified nucleic acid produced by said nucleic acid amplification reaction, and c) determining that said subject is suffering from microbial blood infections when said amount of amplified nucleic acid is higher than that produced in a control sample, wherein said nucleic acid amplification reaction comprises the amplification of the *tuf* gene or *tuf* gene product, or a part thereof, of *Staphylococcus* spp., preferably wherein said nucleic acid amplification reaction is a PCR reaction using as a forward primer the sequences 5'-CCAACTCCAGAACGTGATTCTG-3', 5'-CCAACTCCAGAACGTGACTCTG-3' and 5'-CCAACACCAGAACGTGATTCTG-3', using as reverse primers the sequences 5'-GTTGTCACCAGCTTCAGCGTAGT-3', 5'-GTTATCACCAGCTTCAGCGTAAT-3', and 5'-GTTGTCACCAGCTTCAGCATAGT-3', and using as a probe the sequence 5'-ACAGGCCGTGTTGAACGTGGKCAAATCAA-3'.

A method of the invention preferably further comprises the amplification of a gene or gene product, or a part thereof, of at least one microorganism selected from the group consisting of *Enterococcus faecalis, Escherichia coli,* and *Staphylococcus aureus,* as part of the same or a separate nucleic acid amplification reaction.

In one embodiment of a method of the invention, the subject is a neonate, and a method of the invention further comprises the amplification of a gene or gene product, or a part thereof, of at least one microorganism selected from the group consisting of *Streptococcus agalactiae* and *Serratia marcescens,* as part of the same or a separate nucleic acid amplification reaction.

A preferred method for neonatal analysis comprises performing 3 separate multiplex PCR assays, wherein a first amplification reaction comprises the amplification of a gene or gene product, or a part thereof, of *Staphylococcus aureus, Enterococcus faecalis, Klebsiella* spp., and *Streptococcus agalactiae*; wherein a second amplification reaction comprises the amplification of a gene or gene product, or a part thereof, of *Escherichia coli, Pseudomonas aeruginosa,* and *Serratia marcescens*; and wherein a third amplification reaction comprises the amplification of a gene or gene product, or a part thereof, of *Staphylococcus* spp.

Said gene of *Serratia marcescens* preferably is the *gyrB* gene and said nucleic acid amplification reaction is a real-time PCR reaction using as a forward primer the sequence 5'-GACCGTGAAGACCACTTCCATTAC-3', , using as reverse primers the sequences 5'-ACGCCGATGTCGTCTTTCAC-3' and 5'-CACGCCGATATCGTCTTTCAC-3', and using as a probe the sequence 5'-CGATCCACCCGAACGTGTTCTACTTCTC-3'.

Said gene of *Enterococcus faecalis* preferably is the 16S rRNA gene and said nucleic acid amplification reaction is a real-time PCR reaction using as a forward primer the sequence 5'-CGCTTCTTTCCTCCCGAGT-3', using as reverse primer the sequence 5'-GCCATGCGGCATAAACTG-3', and using as a probe the sequence 5'-CAATTGGAAAGAGGAGTGGCGGACG-3'.

Said gene of *Escherichia coli* preferably is the 16S rRNA gene and said nucleic acid amplification reaction is a real-time PCR reaction using as a forward primer the sequence 5'-CATGCCGCGTGTATGAAGAA-3', using as reverse primer the sequence 5'-CGGGTAACGTCAATGAGCAAA-3', and using as a probe the sequence 5'-TATTAACTTTACTCCCTTCCTCCCCGCTGAA-3'.

Said gene of *Staphylococcus aureus* preferably is the *Sa442* genetic fragment and said nucleic acid amplification reaction is a real-time PCR reaction using as a forward primer the sequence 5'-CATCGGAAACATTGTGTTCTGTATG-3', using as reverse primer the sequence 5'-TTTGGCTGGAAAATATAACTCTCGTA-3', and using as a probe the sequence 5'-AAGCCGTCTTGATAATCTTTAGTAGTACCGAAGCTGGT-3'.

Said gene of *Streptococcus agalactiae* preferably is the *cfb* gene and said nucleic acid amplification reaction is a real-time PCR reaction using as a forward primer the sequence 5'-TTCACCAGCTGTATTAGAAGTACATGC-3', using as reverse primer the sequence 5'-CCCTGAACATTATCTTTGATATTTCTCA-3', and using as a probe the sequence 5'-CAAGCCCAGCAAATGGCTCAAAAGCT-3'.

In a further embodiment of a method of the invention, especially wherein the subject is an non-neonate, preferably an adult, a method involving amplification of the *phzE* gene or *phzE* gene product, or a part thereof, of *Pseudomonas aeruginosa,* amplification of the *rhaA* gene or *rhaA* gene product, or a part thereof, of *Klebsiella* spp.,and/or amplification of the *tuf* gene or gene product, or a part thereof, of *Staphylococcus* spp., amplification of a gene or gene product, or a part thereof, of at least one microorganism selected from the group consisting of *Enterococcus faecalis, Escherichia coli,* and *Staphylococcus aureus,* further comprises the amplification of a gene or gene product, or a part thereof, of at least one of the following targets: a microorganism selected from the group consisting of *Acirtetobacter baumannii, Enterococcus faecium, Streptococcus pneumoniae, Enterococcus* spp., *Candida* spp., *Candida albicans, Candida glabrata, Candida krusei, Aspergillus* spp, Gram positive bacteria, Gram negative bacteria, or a gene or gene product selected from mecA, vanA, and ctxM as part of the same or separate nucleic acid amplification reactions.

This method preferably comprises performing 5 separate multiplex PCR assays, wherein a first amplification reaction comprises the amplification of a gene or gene product, or a part thereof, of *Aspergillus* spp, Gram positive bacteria, Gram negative bacteria, *Candida* spp. and *Candida glabrata*; wherein a second amplification reaction comprises the amplification of a gene or gene product, or a part thereof, of *Escherichia coli, Enterococcus faecium, Acinetobacter baumannii,* and the *mecA* gene; wherein a third amplification reaction comprises the amplification of a gene or gene product, or a part thereof, of *Staphylococcus aureus, Enterococcus faecalis, Pseudomonas aeruginosa, Candida krusei,* and *Streptococcus pneumoniae*; wherein a fourth amplification reaction comprises the amplification of a gene or gene product, or a part thereof, of *Staphylococcus* spp., *Enterococcus* spp., *Klebsiella* spp. and *Candida albicans,* and wherein a fifth amplification reaction comprises the amplification of a gene or gene product, or a part thereof, of *vanA* and *ctxM,* preferably *ctxM-1* and/or *ctxM-9.*

In this embodiment, said gene of *Escherichia coli* preferably is the *gadA* and/or *gadB* gene and said nucleic acid amplification reaction is a real-time PCR reaction using as a forward primer the sequence 5'-GGCTTCGAAATGGACTTTGCT-3', using as reverse primer the sequence 5'-TGGGCAATACCCTGCAGTTT-3', and using as a probe the sequence 5'-CTGTTGCTGGAAGACTACAAAGCCTCCCTG-3'.

Said gene of *Acinetobacter baumannii* preferably is the 23S rDNA gene and said nucleic acid amplification reaction is a real-time PCR reaction using as a forward primer the sequence 5'-CGCTGTTGTTGGTGATGGAACT-3', using as reverse primer the sequence 5'-AACAGTTGCAGCGGCCTG-3', and using as a probe the sequence 5'-CTTCCTGAGCTGACGACAGCCGC-3'.

Said gene of *Enterococcus faecium* is a hypothetical protein encoding ORF gene and said nucleic acid amplification reaction is a real-time PCR reaction using as a forward primer the sequence 5'-GCCAAAGGACCGCTTATTACG-3', using as reverse primer the sequence 5'-GCTTTTCGCTGTTTTTTTAATGACT-3', and using as a probe the sequence 5'-TTCGCAAGCGACAACAAGCACAAGC-3'.

In this embodiment, said gene of *Staphylococcus aureus* is the hsdM gene and said nucleic acid amplification reaction is a real-time PCR reaction using as a forward primer the sequence 5'-AAGGCGGAGGAATCACATGTC-3', using as reverse primer the sequence 5'-TTCGCAATCGACCATAATTTTTT-3', and using as a probe the sequence 5'-TTACTGAAAAACAACGTCAGCA-3'.

In this embodiment, said gene of *Enterococcus faecalis* preferably is the *ref12A* gene wherein said nucleic acid amplification reaction is a real-time PCR reaction using as a forward primer the sequence 5'-ATGCGTCTCGTCACAGTA-3', using as reverse primer the sequence 5'-GGTACGATGATTTCATCTGT-3', and using as a probe the sequence 5'-AGTTGCGATGTTTCACTGTGAAGCA-3'.

Said gene of *Streptococcus pneumoniae* preferably is the *comX* gene and said nucleic acid amplification reaction is a real-time PCR reaction using as a forward primer the sequence 5'-GGTCTCTGGCTAGATGATTATTATCTCTT-3', using as reverse primer the sequence 5'-ATAGTAAACTCCTTAAACACAATGCGTAA-3', and using as a probe the sequence 5'-CGCCCTCGAAATCGTTCATTGCTTAAGA-3'.

Said gene of *Aspergillus* spp preferably is the 18S-28S rRNA ITS region and said nucleic acid amplification reaction is a real-time PCR reaction using as a forward primer the sequence 5'-GCGTCATTGCTGCCCTCAAGC-3', using as reverse primer the sequence 5'-ATATGCTTAAGTTCAGCGGGT-3', and using as a probe the sequence 5'-CCTCGAGCGTATGGGGC-3'.

Said gene of Gram positive bacteria preferably is the 16S rDNA gene and said nucleic acid amplification reaction is a real-time PCR reaction using as a forward primer the sequence 5'-TGGAGCATGTGGTTTAATTCGA-3', using as reverse primer the sequence 5'-TGCGGGACTTAACCCAACA-3', and using as a probe the sequence 5'-TGGTGCATGGTTG-3'.

Said gene of Gram negative bacteria preferably is the 16S rDNA gene and said nucleic acid amplification reaction is a real-time PCR reaction using as a forward primer the sequence 5'-TGGAGCATGTGGTTTAATTCGA-3', using as reverse primer the sequence 5'-TGCGGGACTTAACCCAACA-3', and using as a probe the sequence 5'-TGCTGCATGGCTGT-3'.

Said gene of *Candida* spp. preferably is the 18S-28S rRNA ITS region and wherein said nucleic acid amplification reaction is a real-time PCR reaction using as a forward primer the sequence 5'-CATGCCTGTTTGAGCGTCRTTT-3', using as reverse primer the sequence 5'-ATATGCTTAAGTTCAGCGGGT-3', and using as a probe the sequence 5'-TCGTATTGCTCAACACCAAACCC-3'.

Said gene of *Candida glabrata* preferably is the 18S-28S rRNA ITS region and said nucleic acid amplification reaction is a real-time PCR reaction using as a forward primer the sequence 5'-CATGCCTGTTTGAGCGTCRTTT-3', using as reverse primer the sequence 5'-ATATGCTTAAGTTCAGCGGGT-3', and using as a probe the sequence 5'-ATCAGTATGTGGGACACGAGCG-3'.

A nucleic acid amplification reaction comprising said mecA gene or a part thereof preferably is a real-time PCR reaction using as a forward primer the sequence 5'-GATCGCAACGTTCAATTTAATTTT-3', using as reverse primer the sequence 5'-GCTTTGGTCTTTCTGCATTCCT-3', and using as a probe the sequence 5'-AATGACGCTATGATCCCAATCTAACTTCCACAT-3'.

Said gene of *Candida krusei* preferably is the 18S-28S rRNA ITS region and wherein said nucleic acid amplification reaction is a real-time PCR reaction using as a forward primer the sequence 5'-CATGCCTGTTTGAGCGTCRTTT-3', using as reverse primer the sequence 5'-ATATGCTTAAGTTCAGCGGGT-3', and using as a probe the sequence 5'-ACGACGTGTAAAGAGCGTCGG-3'.

Said gene of *Enterococcus* spp. preferably is the 23S rDNA gene and wherein said nucleic acid amplification reaction is a real-time PCR reaction using as a forward primer the sequence 5'-TGCGGGGATGAGGTGTG-3', using as reverse primer the sequence 5'-CAAACAGTGCTCTACCTCCATCAT-3', and using as a probe the sequence 5'-TAGCCCTAAAGCTATTTCGGAGAGAACCA-3'.

Said gene of *Candida albicans* preferably is the 18S-28S rRNA ITS region and wherein said nucleic acid amplification reaction is a real-time PCR reaction using as a forward primer the sequence 5'-CATGCCTGTTTGAGCGTCRTTT-3', using as reverse primer the sequence 5'-ATATGCTTAAGTTCAGCGGGT-3', and using as a probe the sequence 5'-TAAGGCGGGATCGCTTTGACA-3'.

A nucleic acid amplification reaction comprising said *vanA* gene or a part thereof, preferably is a real-time PCR reaction using as a forward primer the sequence 5'-CGGTTTCACGTCATACAGTCGTT-3', using as reverse primer the sequence 5'-CAGTTCGGGAAGTGCAATACC-3', and using as a probe the sequence 5'-TCCCCGTATGATGGCCGCTGC-3'.

A nucleic acid amplification reaction comprising *ctxM-1* gene or a part thereof, preferably is a real-time PCR reaction using as a forward primer the sequence 5'-ATGTGCAGYACCAGTAARGTKATGGC-3', using as reverse primer the sequence 5'-ATCACKCGGRTCGCCNGGRAT-3', and using as a probe the sequence 5'-CCCGACAGCTGGGAGACGAAACGT-3'.

A nucleic acid amplification reaction comprising the *ctxM-9* gene or a part thereof, preferably is a real-time PCR reaction using as a forward primer the sequence 5'-ATGTGCAGYACCAGTAARGTKATGGC-3', using as reverse primer the sequence 5'-ATCACKCGGRTCGCCNGGRAT-3', and using as a probe the sequence 5'-CTGGATCGCACTGAACCTACGCTGA-3'.

The invention further provides a kit of parts adapted for performing a method of the invention, said kit preferably comprising at least one forward or reverse primer or probe as defined herein, preferably wherein at least one of said at least one forward or reverse primer or probe comprises a detectable label, preferably a fluorescent label.

The invention further provides at least one forward or reverse primer or probe as defined herein, preferably wherein at least one of said at least one forward or reverse primer or probe comprises a detectable label, preferably a fluorescent label.

The invention further provides a method for monitoring sepsis or for determining the efficacy of an anti-sepsis treatment, said method comprising performing a method according to the invention on at least two samples, wherein said samples are obtained at different time points in the course of the sepsis or at different time points in the course of an anti-sepsis treatment.

### 4. BRIEF DESCRIPTION OF THE FIGURES

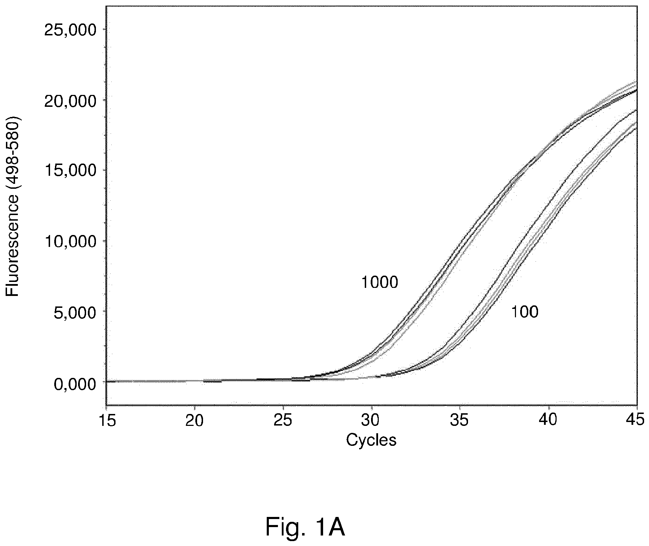

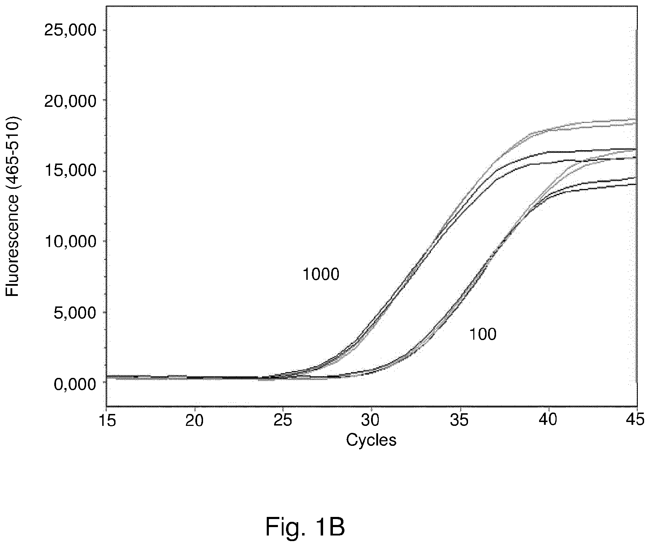

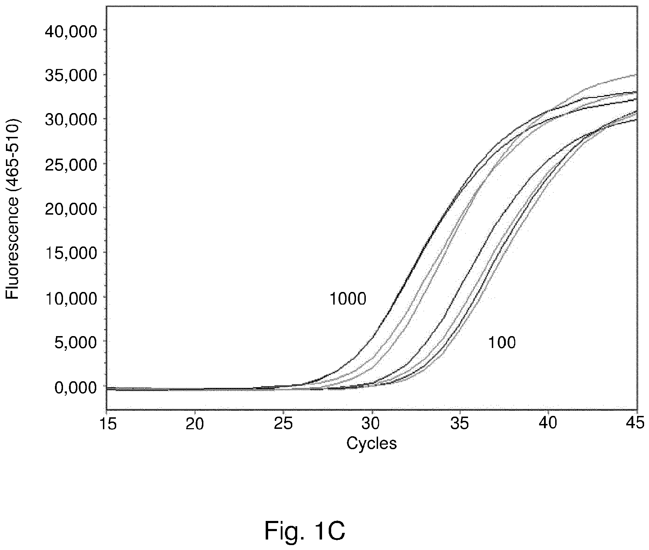

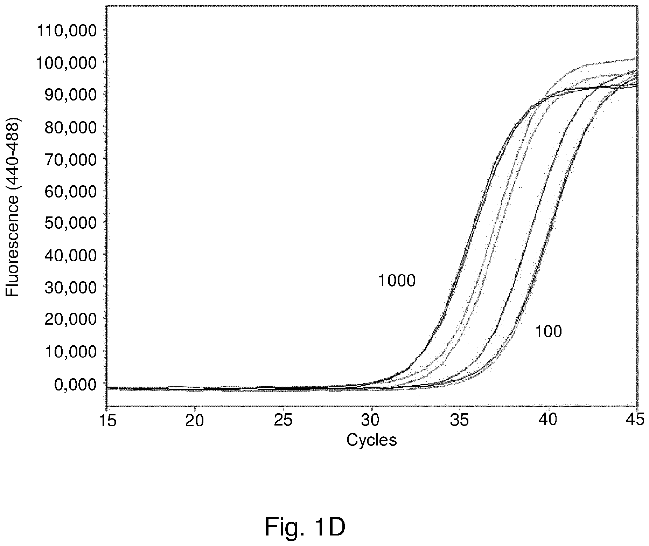

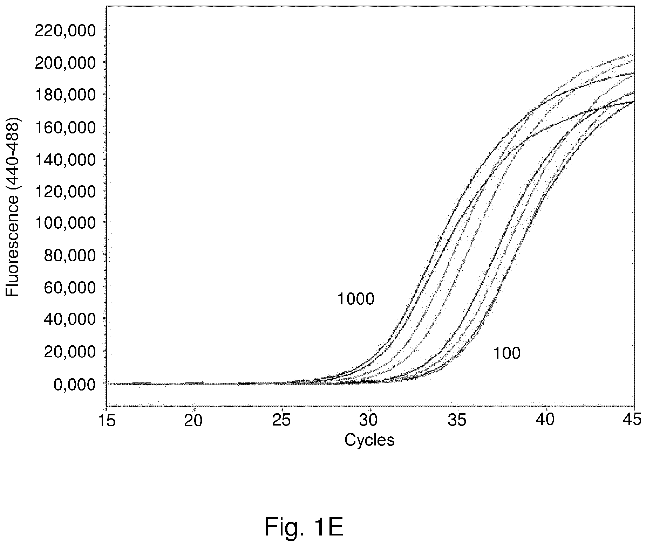

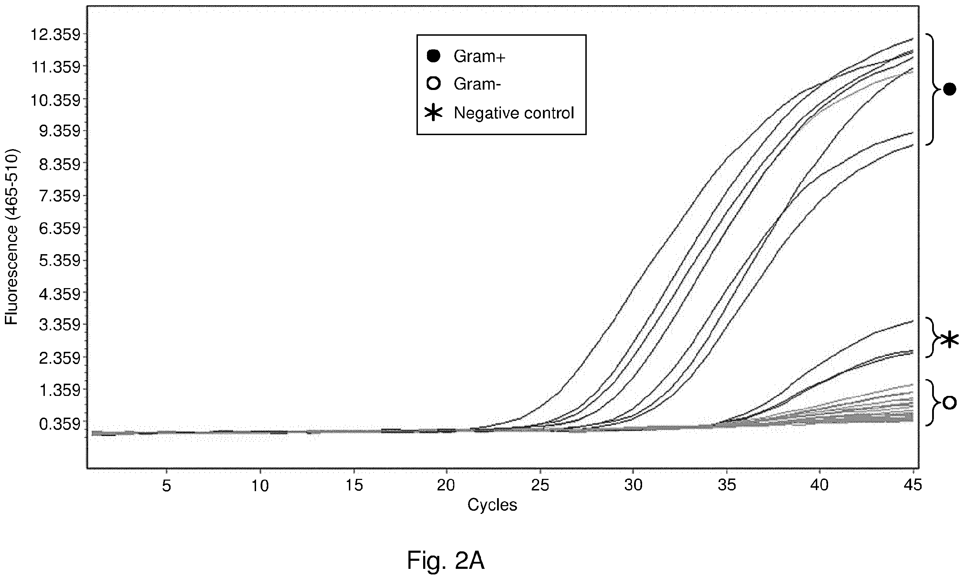

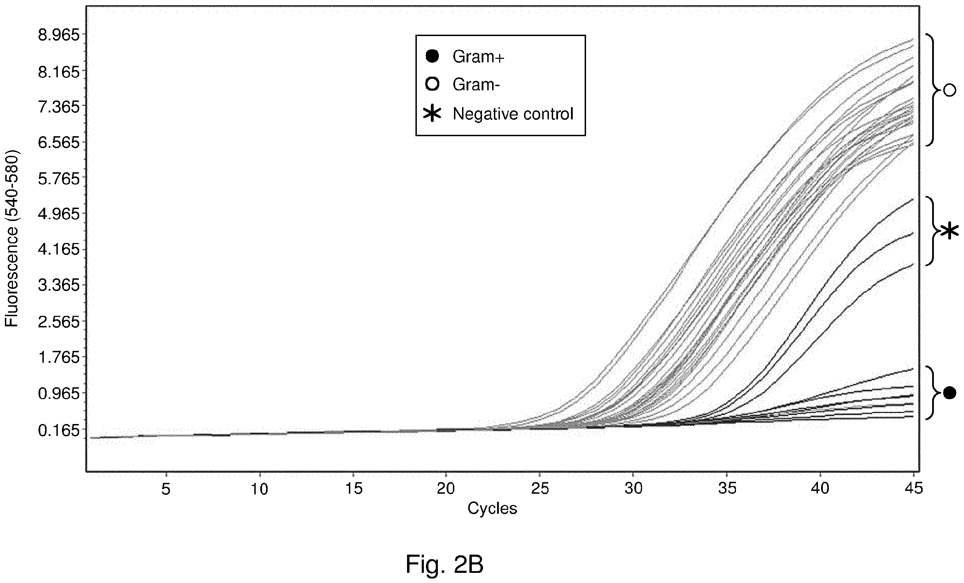

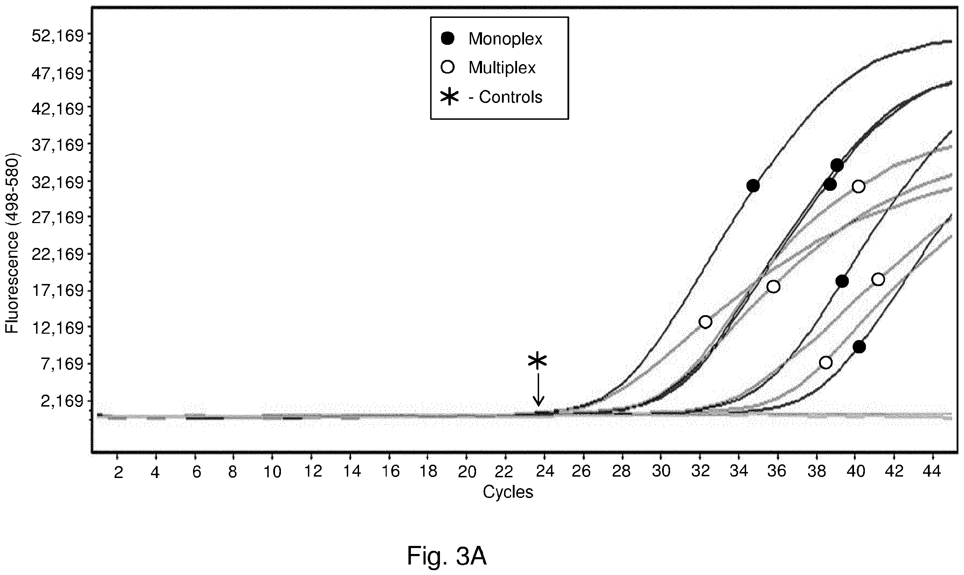

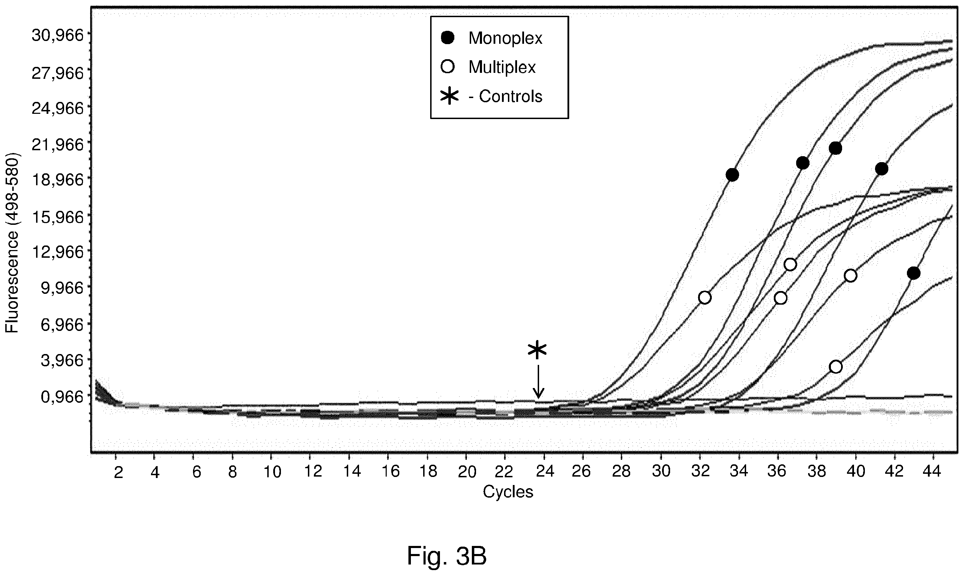

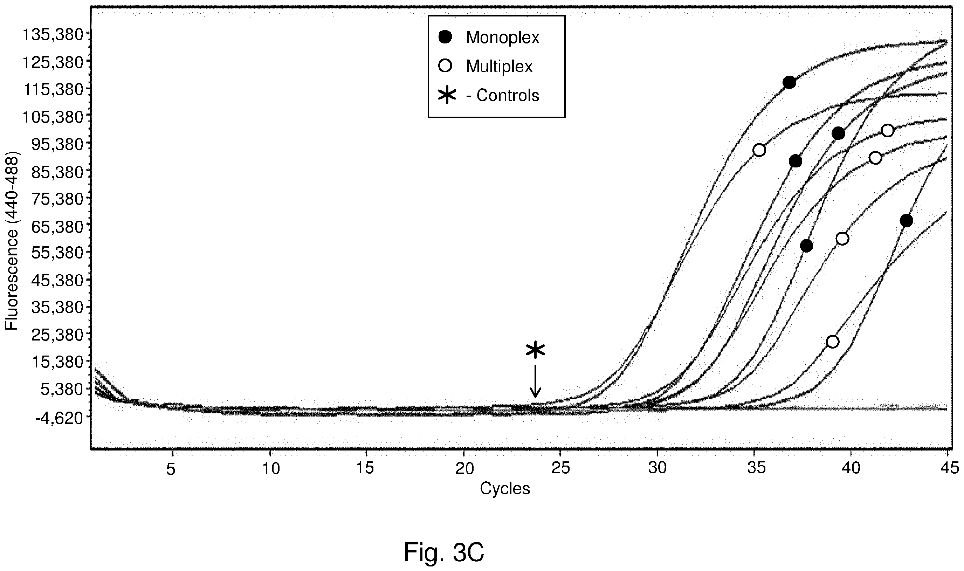

Figure 1 Performance of monoplex PCR assays for detection of DNA from individual bacteria as spiked into blood samples or derived from pure culture and supplied in standard phosphate buffered saline (PBS).
   Bacteria were spiked in two concentrations (1000 and 100 cfu/reaction). Grey curves = bacterial DNA derived from blood. Black curves = bacterial DNA derived from pure culture.
   1A: *Pseudomonas aeruginosa*
   1B: *Klebsiella* genus
   1C: *Enterococcus faecalis*
   1D *Staphylococcus aureus*
   1E: *Staphylococcus* genus
Figure 2 PCRs of Gram-positive and Gram-negative bacteria show background signals (as seen in the curves for the negative control samples) probably due to DNA present in PCR reagents. However, the a-specific signals obtained upon addition of bacterial DNA are always lower than the negative control samples, meaning they will not be unjustly be interpreted as positive.
   2A: Gram-positive PCR
   2B: Gram-negative PCR
Figure 3 Real-time PCR curves, corrected for fluorescence bleed through using the Color Compensation module of software version LightCycler 1.5.0 (Roche) for the indicated micro-organisms. The lowest concentration of pathogen DNA detected in the Multiplex BloodStream Infection (BSI) PCR is indicated in the graphs as cfu/PCR.
   3A: *P. aeruginosa*; Limit of detection: 1 cfu/PCR.
   3B: *Klebsiella* spp.; Limit of detection: 1 cfu/PCR.
   3C: *E. coli;* limit of detection: 1 cfu/PCR.

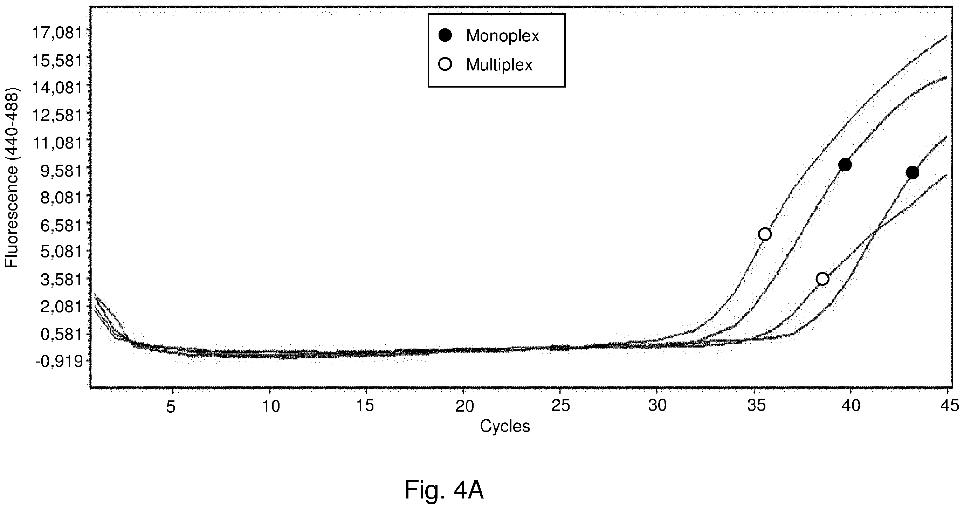

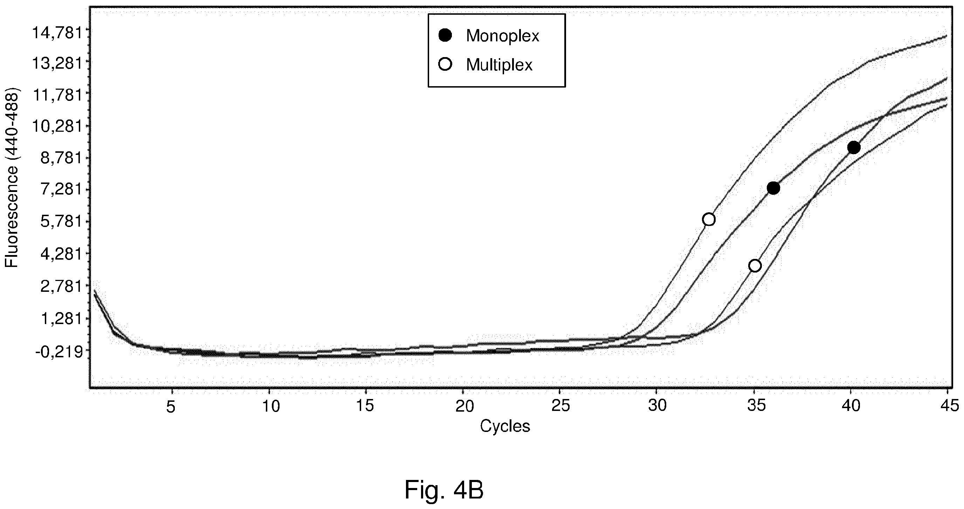

Figure 4 Sensitivity of the pan*-Aspergillus* PCR was tested by adding DNA obtained from pure *Aspergillus* cultures to the PCR reactions. Red curves: monoplex PCR. Blue curves: multiplex PCR.
   4A: *Aspergillus candida*
   4B: *Aspergillus terreus*

### 5. DETAILED DESCRIPTION OF THE INVENTION

### 5.1 Definitions

The term "microbial blood infections", as used herein, refers to the presence of one or more microorganisms in blood of a subject. Said subject not necessarily has clinical signs and/or symptoms of disease. However, the term microbial blood infection includes sepsis.

The term "sepsis", as used herein, refers to an infection-induced syndrome resulting in tissue injury that involves two or more of the following features of systemic inflammation: fever or hypothermia, leukocytosis or leukopenia, tachycardia, and tachypnea or a supranormal minute ventilation. Sepsis is often characterized by signs of inflammation (vasodilation, leukocyte accumulation, increased microvascular permeability) occurring in tissues that are remote from the infection.

The term "blood sample", as is used herein, refers a sample of blood that is obtained from a mammal, preferably a human. The term blood sample includes both blood plasma, which is prepared by removing red and white blood cells, for example by centrifugation, and blood serum, which is prepared by formation of a blood clot, and removal of the clot using, for example, a centrifuge.

The term "control sample" as used herein, refers to a negative control sample, for example a blood sample that does not comprise the indicated microorganism or microorganisms, or a sample of nucleic acids isolated therefrom, or a buffer control, for example 10 mM Tris, pH 8.0, 1 mM ethylenediaminetetraacetic acid (EDTA).

The term "amplification", as is used herein, refers to the *in vitro* amplification of a specific nucleic acid sequence, such as to test for presence of a given fungus or bacteria in a sample. In vitro amplification methods include amplification of a target nucleic acid sequence using, for example, ligase chain reaction (LCR), isothermal ribonucleic acid amplification such as nucleic acid sequence-based amplification (NASBA) and cleavage-based signal amplification of RNA, transcription mediated amplification, strand displacement amplification and, preferably, polymerase chain reaction (PCR).

The term "nucleic acid amplification reaction", as is used herein, refers to a specific amplification reaction or a combination of specific amplification reactions that is performed in a reaction chamber such as a vial or a well of a microtiter plate, or a fluidic chamber of a cartridge system. A nucleic acid amplification reaction is preferably specific, meaning that only the region between two primers in a target nucleic acid template is amplified.

The term "PCR reaction", as is used herein, refers to an amplification reaction that is characterized by repeated cycles of denaturation of target nucleic acid template, annealing of primers, and extension (synthesis) of new nucleic acid strand. The specificity of a PCR reaction is substantially determined by the % identity of the primers to the target nucleic acid template and the annealing temperature.

The term "real-time PCR reaction", as is used herein, refers to a PCR amplification reaction to which a labeled probe or a dye is added to generate a signal. The intensity of the signal is a measure for the amount of product that is generated. Detection of the signal in real-time allows quantification of the amount of starting material. A real-time PCR reaction is performed in specialized thermal cyclers with detection systems that detect the signal, for example a LightCycler 480II (Roche Diagnostics, Almere, The Netherlands), a Mastercycler Realplex Ep Real-Time PCR System (Eppendorf A.G., Hamburg, Germany), or a StepOne™ Plus (Thermo Fisher Scientific Inc., Waltham, MA USA). However, a separate probe does not need to be present. Some real-time PCR reactions incorporate a dye in the primer (e.g. Scorpion® primers; Premier Biosoft, Palo Alto, CA, USA) and are comprised in the scope of the present invention.

The terms "forward primer" and "reverse primer", as are used herein, refer to a single-stranded oligonucleotide or oligonucleotide mimic of 15-50 bases, preferably 16-30 bases, that is complementary to nucleic acid sequences flanking the region to be amplified. The sequence of the forward primer and reverse primer determine the specificity of the amplification reaction. Preferred primers are preferably about 100% identical to a region on a target nucleic acid template such that only the region between two primers in a target nucleic acid template is amplified. The distance between the primer binding sites on the target nucleic acid template will determine the size of the amplified product.

The term "probe", as is used herein, refers to a single-stranded oligonucleotide or oligonucleotide mimic of 15-50 bases, preferably 16-30 bases, that is complementary to a nucleic acid sequence within the region to be amplified. A preferred probe is about 100% identical to a region on a target nucleic acid template.

The terms "target DNA molecule", "target nucleic acid template" and "template DNA molecule", as are used herein, refer to nucleic acid of which a region between two primers, preferably a forward primer and a reverse primer, is amplified. A target nucleic acid template is a gene or a gene product, such as a RNA product, or a part of the gene or part of the gene product.

The term "amplicon", as is used herein, refers to a region on a target nucleic acid template that is amplified using said two primers, preferably a forward primer and a reverse primer. An amplicon preferably comprises a nucleic acid sequence that is complementary to a nucleic acid sequence of a probe that specifically recognizes said amplicon.

The term "Ct value", as used herein, refers to the cycle threshold in real time PCR, which is the number of cycles required for a fluorescent signal to cross a background level. Ct levels are inversely proportional to the amount of target nucleic acid in the sample, meaning that a lower Ct level indicates a higher amount of target nucleic acid template in the sample. In general, for a real time PCR assay undergoing 40 cycles of amplification, a Ct value of < 29 is indicative of abundant target nucleic acid template in a sample, a Ct value of 30-37 is indicative of a moderate amount of target nucleic acid template, while a Ct value of 38-40 is indicative of a low amount of target nucleic acid template.

The term "gene", as is used herein, refers to genomic DNA sequence that encodes a gene product. The term "gene" includes enhancer, promoter, intronic and exonic sequences.

The term "gene product", as is used herein, refers to a nucleic acid product, preferably a messenger RNA, that is encoded by a gene. When a gene product such as RNA is to be amplified, the RNA is preferably converted into copy DNA by a RNA-dependent DNA polymerase, also termed reverse-transcriptase, prior to amplification.

The term "oligonucleotide mimic", as is used herein, refers to a modified oligonucleotide comprising, for example, locked nucleic acid (LNA®), synthetic peptide nucleic acid, mimics comprising 2'O-Me modified nucleotides or phosphonoacetate modified oligonucleotides, and/or mimics comprising phosphodiester-, phosphonocarboxylate-, methylphosphonate- or phosphorothioate internucleotide bonds.

The term "detectable label", as is used herein, refers to a label that is detectable by spectroscopic, photochemical, biochemical, immunochemical, or chemical means. For example, useful labels include radioactive labels, fluorescent labels, electron-dense reagents, enzymes (as commonly used in ELISAs), biotin, or haptens and proteins for which antisera or monoclonal antibodies are available.

The term "colony forming unit-equivalent", as is used herein, refers to an amount of a micro-organism that is detected in a real-time PCR reaction according to the invention, when compared to a standard blood culture test. A cell of a micro-organism will in principle give rise to a colony in an appropriate standard blood culture test, and detection of this amount of a micro-organism in a real-time PCR reaction according to the invention will result in detection of a colony forming unit-equivalent.

The term "multiplex PCR assay", as is used herein, refers to the simultaneous amplification of different nucleic acid fragments in a single amplification reaction.

The term "separate multiplex PCR assays", as is used herein, refers to the amplification of different nucleic acid fragments in multiple separate amplification reactions.

The term "neonate", as is used herein, refers to a mammal, preferably a human, preferably up to three months after birth.

The term "non-neonate", as is used herein, refers to a mammal, preferably human, that is older than three months after birth. A preferred non-neonate is an adult of 18 years or older.

### 5.2 Sample preparation

Early detection of the causing microorganism and timely therapeutic intervention are crucial for improved outcome of patients with microbial blood infections such as sepsis. The early detection of specific pathogens will allow the discrimination between patients with SIRS and patients with microbial blood infections such as sepsis, and will have a positive impact on therapy. The present inventors have developed Polymerase Chain Reaction (PCR)-based tests that identify microorganisms and provide information about their resistance to antibiotics. The tests can be applied to biological fluids, particularly blood samples.

Efforts to introduce molecular methods for diagnosis of microbial blood infections such as sepsis, especially neonatal sepsis, have evolved around broad-range PCR targeting the 16S rRNA gene for universal bacterial or gram-specific detection (McCabe et al. 1995. Pediatrics 95: 165-9; Jordan et al. 2006. J Mol Diagn 8: 357-63; Wu et al. 2008. J Clin Microbiol 46: 2613-9.; Chan et al. 2009. Crit Care Med 37: 2441-7). A recent systematic review with meta-analysis evaluating these assays showed a mean sensitivity of 90% and specificity of 96% compared to blood culture indicating that such assays currently offer a promising potential as add-on tests (Pammi et al. 2011. Pediatrics 128:e973-85).

However, an important limitation of these broad-range assays is that additional processing steps such as sequencing or hybridization are required for species identification thereby prolonging the turnaround time significantly. Also, they are particularly prone to false-positive results coming from exogenous bacterial DNA present in PCR reaction components (e.g. Taq polymerase preparation) or from contamination during processing steps. These issues reduce both sensitivity and applicability of these assays (Corless et al. 2000. J Clin Microbiol 38:1747-52). A species-specific PCR assay could overcome these drawbacks, but should be applied in a multiplexed format to provide sufficient coverage of etiologic pathogens. The implementation of real-time multiplex assays in the diagnostic process is rapidly growing and recent studies report sensitivities equal to monoplex assays (Wittwer et al. 2001. Methods 25:430-42; Molenkamp et al. 2007. J Virol Methods 141:205-11; Bahrdt et al. 2010. Anal Bioanal Chem 396: 2103-1). As such, these type of assays may as well provide a good option for diagnosis of microbial blood infections such as neonatal sepsis.

Therefore, an easy-to-use, fast and sensitive multiplex PCR assay was developed that detects the most prevalent bacterial pathogens in a species-specific manner and which requires only small input blood volumes

Blood samples are preferably collected in tubes that are coated with anticoagulants such as EDTA, sodium citrate, and heparin. Heparin is preferably avoided when RNA is isolated, because heparin may inhibit the synthesis of copy DNA by a reverse transcriptase. Preferred amounts of biological fluids, particularly blood samples, that are collected are between 0.1 and 10 ml, preferably between 1 and 5 ml.

The biological fluid, preferably blood sample, is preferably pre-treated to induce selective lysis of human cells and degradation of non-target human DNA, which results in increased sensitivity and specificity of the subsequent analysis of pathogens. Kits for the removal of human cells and the degradation of non-target human DNA are known in the art. Very suitable systems have been described, such as the Polaris system (WO2011/070507 and WO2012/168003) and/or are commercially available, such as the MolYsis pretreatment kit (Molzym GmbH & Co. KG, Bremen, Germany). These methods allow larger sample volumes to be processed. This will result in increased sensitivity, rendering the molecular detection of bloodstream infections clinically applicable. The Polaris method is preferred as it is more reproducible, less labour intensive, and faster compared to the MolYsis method.

PCR is a process in which DNA sample is amplified. DNA samples may be obtained by using generally known techniques for DNA isolation. Methods and compositions for isolation of genomic DNA from biological fluids, particularly blood, preferably employ aqueous solvents without use of organic solvents and chaotropic salts. The isolated genomic DNA is preferably free of polymerase inhibitors.

Total genomic DNA may be purified from blood samples, or from pretreated blood samples as is indicated herein above, using, for instance, a combination of physical and chemical methods. Very suitably commercially available systems for DNA isolation are used, such as the QIAamp blood mini kit columns (Qiagen, Venlo, The Netherlands), the NucliSENS® easyMAG® nucleic acid extraction system (bioMérieux, Marcy l'Etoile, France) or the MagNA Pure 96 System (Roche Diagnostics, Almere, The Netherlands).

As an alternative, a gene product such as ribonucleic acid (RNA) may be isolated from a blood sample, or from a pretreated blood sample as is indicated herein above, using, for example, the Ambion Ribopure™ kit or the Qiagen RNeasy kit. RNA is preferably converted into complementary DNA (cDNA) prior to amplification using a RNA-dependent DNA polymerase or reverse transcriptase. Methods for the conversion of RNA into cDNA are known in the art and include recombinant M-MuLV reverse transcriptase or AMV reverse transcriptase. Suitable commercially available systems for cDNA synthesis include commercially available systems for DNA isolation are used, such as the qScript™ cDNA synthesis kit (Quanta Biosciences, Gaithersburg, MD) and the Maxima H Minus First Strand cDNA Synthesis Kit (Thermo Fisher Scientific Inc., Waltham, MA). It is preferred that random primers, for example hexamers or nonamers, or gene-specific primers are used for cDNA synthesis.

### 5.3 Amplification reaction

Different amplification methods, known to a skilled artisan, can be employed for amplification, including but not limited to Polymerase Chain Reaction (PCR), rolling circle amplification, nucleic acid sequence-based amplification, transcription mediated amplification, and linear RNA amplification. A preferred amplification method is PCR, especially real-time PCR.

PCR is a technology that relies on thermal cycling, consisting of cycles of repeated heating and cooling of a reaction for DNA melting and enzymatic replication of the DNA. Primers containing sequences that specifically hybridizes to the target region, and a DNA polymerase are key components to enable selective and repeated amplification. As PCR progresses, the amplified DNA product that is generated is itself used as a template for replication, resulting in a chain reaction in which the DNA template is exponentially amplified.

A preferred DNA polymerase is a thermo stable polymerase, preferably a thermo stable recombinant polymerase. Preferred commercially available DNA polymerases include AptaTaq Fast DNA Polymerase and LightCycler® FastStart Enzyme (Roche Diagnostics, Almere, The Netherlands).

Real-time PCR, also called quantitative PCR (qPCR), is a technique which is used to amplify and simultaneously quantify a template DNA molecule. The detection of the amplification products can in principle be accomplished by any suitable method known in the art. The amplified products may be directly stained or labelled with radioactive labels, antibodies, luminescent dyes, fluorescent dyes, or enzyme reagents. Direct DNA stains include for example intercalating dyes such as acridine orange, ethidium bromide, ethidium monoazide or Hoechst dyes.

Alternatively, the amplified product may be detected by incorporation of labelled dNTP bases into the synthesized DNA fragments. Detection labels which may be associated with nucleotide bases include, for example, fluorescein, cyanine dye and BrdUrd.

When using for example Scorpion primers or a probe-based detection system, a primer or the probe is preferably labelled with a detectable label, preferably a fluorescent label. Preferred labels for use in this invention comprise fluorescent labels, preferably selected from Atto425 (ATTO-TEC GmbH, Siegen, Germany), Atto 647N (ATTO-TEC GmbH, Siegen, Germany), YakimaYellow (Epoch Biosciences Inc, Bothell, WA, USA), Cal610 (BioSearch Technologies, Petaluma, CA, USA), Cal635 (BioSearch Technologies, Petaluma, CA, USA), FAM (Thermo Fisher Scientific Inc., Waltham, MA USA), TET (Thermo Fisher Scientific Inc., Waltham, MA USA), HEX ((Thermo Fisher Scientific Inc., Waltham, MA USA), cyanine dyes such as Cy5, Cy5.5, Cy3, Cy3.5, Cy7 (Thermo Fisher Scientific Inc.,

Waltham, MA USA), Alexa dyes (Thermo Fisher Scientific Inc., Waltham, MA USA), Tamra (Thermo Fisher Scientific Inc., Waltham, MA USA), ROX (Thermo Fisher Scientific Inc., Waltham, MA USA), JOE (Thermo Fisher Scientific Inc., Waltham, MA USA), fluorescein isothiocyanate (FITC, Thermo Fisher Scientific Inc., Waltham, MA USA), and tetramethylrhodamine (TRITC, Thermo Fisher Scientific Inc., Waltham, MA USA). A probe is preferably labeled at the 5' end with a detectable label, preferably a fluorescent label.

A primer such as a Scorpion primer, or a probe preferably has a fluorescent label at one end and a quencher of fluorescence at the opposite end of the probe. The close proximity of the reporter to the quencher prevents detection of its fluorescence; breakdown of the probe by the 5' to 3' exonuclease activity of polymerase breaks the reporter-quencher proximity and thus allows unquenched emission of fluorescence, which can be detected after excitation with a laser. An increase in the product targeted by the reporter probe at each PCR cycle therefore causes a proportional increase in fluorescence due to the breakdown of the probe and release of the reporter. Quenchers, for example tetramethylrhodamine TAMRA, dihydrocyclopyrroloindole tripeptide minor groove binder, are known in the art.

Preferred quenchers are Black Hole Quencher®-1 (BHQ1) and BHQ2, which are available from Biosearch Technologies, Petaluma, CA, USA). The BHQ1 dark quencher has strong absorption from 480 nm to 580 nm, which provides excellent quenching of fluorophores that fluoresce in this range, such as FAM, TET, CAL Fluor® Gold 540, JOE, HEX, CAL Fluor Orange 560, and Quasar® 570 dyes. The BHQ2 dark quencher has strong absorption from 599 nm to 670 nm, which provides excellent quenching of fluorophores that fluoresce in this range, such as Quasar® 570, TAMRA, CAL Fluor® Red 590, CAL Fluor Red 610, ROX, CAL Fluor Red 635, Pulsar® 650, Quasar 670 and Quasar 705 dyes. BHQ1 and BHQ2 may quench fluorescence by both FRET and static quenching mechanisms.

The term "specifically hybridizing" refers to a nucleic acid molecule that is capable of hybridizing specifically under stringent hybridization conditions to a target nucleic acid template that is obtained or derived from *Pseudomonas aeruginosa, Klebsiella species, Escherichia coli, Acinetobacter baumannii, Enterococcus faecalis, Enterococcus faecium, Staphylococcus species, Staphylococcus aureus, Streptococcus pneumoniae, Streptococcus agalactiae, Serratia marcescens, Candida albicans, Candida glabrata, Candida krusei,* Pan*-Aspergillus,* pan*-Candida,* Gram-positive bacteria, Gram-negative bacteria, MecA, VanA, CTXM-1, and/or CTXM-9.

The terms "stringency" and "stringent hybridization" refer to hybridization conditions that affect the stability of hybrids, e.g., temperature, salt concentration, pH, and the like. These conditions are empirically optimised to maximize specific binding and minimize non- specific binding of primer or probe to its target nucleic acid sequence. The terms as used include reference to conditions under which a probe or primer will hybridize to its target sequence, to a detectably greater degree than other sequences (e.g. at least 2-fold over background). Stringent conditions may be sequence dependent and will be different in different circumstances. Longer sequences hybridise specifically at higher temperatures. Generally, stringent conditions are selected to be about 5°C lower than the thermal melting point (Tm) for the specific sequence at a defined ionic strength and pH. The Tm is the temperature (under defined ionic strength and pH) at which 50% of a complementary target sequence hybridises to a perfectly matched probe or primer. Hybridization procedures are well known in the art and are described by e.g. Ausubel et al., 1998. Current Protocols in Molecular Biology, John Wiley, New York; and Sambrook et al., 2001. Molecular cloning: a laboratory manual. Cold Spring Harbor Laboratory Press, New York.

An oligonucleotide primer or probe, or an oligonucleotide mimic primer or probe, is able to hybridize to a target nucleic acid template when the length of the molecule is or resembles at least 15 bases. The length of the primer or probe is preferably less than 100 bases. A preferred length of a primer or probe is between 15 and 50 bases, preferably between 16 and 30 bases.

In general, a primer or probe is able to hybridize to a target nucleic acid template when the percentage of sequence identity of the molecule is at least 90% over substantially the whole length, more preferred at least 91%, more preferred at least 92%, more preferred at least 93%, more preferred at least 94%, more preferred at least 95%, more preferred at least 96%, more preferred at least 97%, more preferred at least 98%, more preferred at least 99%, more preferred 100% identical to a nucleic acid that is obtained or derived from said target nucleic acid template over substantially the whole length of the primer or probe. The term "substantially the whole length" is used to indicate that the probe may comprise additional nucleotide sequences, for example at the 5' and/or 3' ends that are not present in the gene or region described herein above.

Efficient real-time PCR reactions are dependent upon high quality primer and probe design. Rules of thumbs for the design of primers include the selection of primers having a Tm between 58°C and 65°C while keeping the annealing temperatures of the primers as close as possible, having no more than two G's or C's in the last 5 bases at the 3' end, and the selection of primer pairs with minimal number of potential primer dimers and primer hairpins.

Rules of thumbs for the design of probes include the selection of probes that have a Tm between 68°C and 72°C, have no Gs on the 5' end, resemble a strand that has more C than G bases, and are as short as possible, without being shorter than 13 nucleotides.

Methods for the design of primers and probes are known in the art. For example, Premier Biosoft (Palo Alto, CA, USA) offers AlleleID® and Beacon Designer™ to design probes for real-time PCR assays that are free of dimers, repeats and runs and ensure signal fidelity. In addition, Primer3 (http://primer3.sourceforge.net) and Integrated DNA Technologies, Inc. (www.idtdna.com/Scitools/Applications/Primerquest) provide online tools for the design of primers and probes for real-time PCR assays. Hence, the skilled person is able to design primers and probes for real-time PCR analyses of one or more target nucleic acid templates.

The primers and probes are preferably tested in single nucleic acid amplification reactions (monoplex) and combined nucleic acid amplification reactions (multiplex) to determine optimal combinations of specific nucleic acid amplification reactions.

Specific tests have been developed for the identification of species and resistance markers based on their prevalence in blood cultures of patients suffering from sepsis. Genus- and species-specific real-time PCR assays were developed using MultiMPrimer3 (available at http://bioinfo.ut.ee/multimprimer3/) or from alignments of gene sequences retrieved from GenBank (Koressaar et al., 2009. Bioinformatics 25: 1349-55). Sequences with *adequate in silico* coverage and specificity were used for primer and probe design for real-time PCR assays using Primer Express 149 Software 3.0 (Life Technologies, Bleiswijk, The Netherlands).

Optimized primers and probes for multiplex real-time PCR are provided in Tables 1 and 2. The most common infections underlying microbial blood infections such as sepsis are caused by *Pseudomonas aeruginosa, Escherichia coli, Klebsiella species, Enterococcus faecalis, Staphylococcus* spp. and *Staphylococcus aureus.*

A preferred test for microbial blood infections such as sepsis comprises detection of the *phzE* gene or *phzE* gene product, or a part thereof, of *Pseudomonas aeruginosa,* the *rhaA* gene or *rhaA* gene product, or a part thereof, of *Klebsiella spp.,* and/or the *tuf* gene or *tuf gene* product, or a part thereof of *Staphylococcus* spp..

The term *phzE* gene refers to a gene of which the encoded protein product plays a role in the biosynthesis of pyocyanin and phenazine-1-carboxamide from chorismic acid. PhzE catalyses the transfer of an amide nitrogen from glutamine to chorismic acid, creating 2-amino-2-deoxyisochorismic acid. PhzE resembles a class I glutamine amidotransferase (GATase). PhzE is part of a seven-gene phenazine biosynthetic locus comprising the operons phzABCDEFG. A complete sequence of this locus is provided, for example, in GenBank accession number AF005404.2. Preferred primers and probe are indicated in Table 1 and Table 2.

The term rhaA gene, as used herein, refers to a gene that encodes a rhamnulose-1-phosphate aldolase (EC 4.1.2.19), which catalyzes the chemical reaction L-rhamnulose 1-phosphate ↔ glycerone phosphate + (S)-lactaldehyde. A sequence of this gene of Klebsiella spp. is provided, for example, in GenBank accession number CP006923.1. Preferred primers and probe are indicated in Table 1 and Table 2.

Said *tuf* gene of *Staphylococcus* spp., is a gene of *Staphylococcus* spp. that encodes an elongation factor EF-Tu, which is involved in the elongation phase of polypeptide synthesis on ribosomes. The coding region of the *tuf* gene is located in a genomic region between nucleotides 580338 and 581522 of the complete genome of *Staphylococcus aureus* subsp. aureus SA957, as provided in GenBank accession number CP003603.1. Preferred primers and probes are indicated in Table 1 and Table 2.

A further preferred test comprises detection of the *phzE* gene or *phzE* gene product, or a part thereof, of *P. aeruginosa,* the rhaA gene or rhaA gene product, or a part thereof, of *Klebsiella* spp, detection of Staphylococcus spp., preferably by detection of a tuf gene, and detection of *Enterococcus faecalis,* preferably by detection of a 16S rRNA gene or of a *ref12A* gene, detection of *Escherichia coli,* preferably by detection of a 16S rRNA gene or of a *gad* and/or *gadB* gene, and detection of *Staphylococcus aureus,* preferably by detection of a *SA442* gene or of a hsdM gene.

The 16S rRNA gene of *E. faecalis* and the 16S rRNA gene of *E. coli* encode a 16S ribosomal RNA (or 16S rRNA), which is a component of the 30S small subunit of prokaryotic ribosomes. The 16S rRNA gene is highly conserved between different species of bacteria. 16S rRNA gene sequences contain hypervariable regions that can provide species-specific signature sequences useful for bacterial identification. The Ribosomal Database Project (RDP) provides annotated ribosomal rRNA sequences along with related programs and services and is available at http://rdp.cme.msu.edu. Preferred primers and probe sequences are indicated in Table 1.

Said *ref12A* gene of *Enterococcus faecalis* is a member of a complex family of palindromic repeat sequences that is located, for example, between nucleotides 690164 and 690257, between nucleotides 1382699 and 1382792, between nucleotides 1755360 and 1755453, and between nucleotides 2895546 and 2895639 of the genomic sequence of *Enterococcus faecalis* DENG1, as depicted in GenBank accession number CP004081.1. Preferred primers and probe are indicated in Table 2.

Said *gadA* and/or *gadB* gene of *E. coli,* is a gene that encodes 2 biochemically identical isoforms of glutamate decarboxylase (GAD). Coding sequences of the *gadA* and *gadB* genes of *E. coli* strain DEC 1a are provided in GenBank accession numbers EF547386.1 and EF551351.1, respectively. These two sequences are 86% identical over the complete nucleotide sequences, with subregions showing 100% identity. Preferred primers and probe are indicated in Table 2.

Said *SA442* gene of *S. aureus* is a genetic fragment that encodes a glutamate synthase domain-containing putative membrane protein. The gene is located between nucleotides 2685975 to 2687552 in the genomic sequence *of S. aureus* subsp. aureus Z172 having GenBank accession numberCP006838.1. Preferred primers and probes are indicated in Table 1.

Said *hsdM* gene of *S. aureus* is a gene with locus tag ER16_01940 that encodes a site-specific DNA-methyltransferase activity. This gene is located, for example, in a genomic region between nucleotides 437403 and 438959 of the genomic sequence of *S. aureus* subsp. aureus strain H-EMRSA-15, as depicted in GenBank accession number CP007659.1. Preferred primers and probes are indicated in Table 2.

Neonatal sepsis is the single most important cause of neonatal deaths, accounting for over 50% of all incidences, especially in preterm infants. If diagnosed early and treated aggressively with antibiotics and good supportive care, it is possible to save most cases of neonatal sepsis. Late-onset septicemia is caused by the organisms thriving in the external environment of the home or the hospital. The infection is often transmitted through the hands of care-providers, resulting in late onset sepsis (LOS), whereby the onset of symptoms is usually delayed beyond 72 hours after birth. The associated factors of LOS include low weight, lack of breastfeeding, superficial infections, aspiration of feeds, disruption of skin integrity with needle pricks and use of intravenous fluids. A preferred assay detects the eight most prevalent bacterial pathogens that cause LOS in preterm and very low birth weight (VLBW) infants.

A preferred neonatal sepsis test comprises detection of the *phzE* gene or *phzE* gene product, or a part thereof, of *P. aeruginosa,* the *rhaA* gene or rhaA gene product, or a part thereof, of *Klebsiella* spp, and detection of *Enterococcus faecalis,* preferably by detection of a 16S rRNA gene, *Escherichia coli,* preferably a 16S rRNA gene, *Staphylococcus* spp., preferably a *tuf* gene, *Staphylococcus aureus,* preferably a *SA442* gene, *Streptococcus agalactiae,* preferably a *cfb* gene, and *Serratia marcescens,* preferably a *gyrB* gene. This assay has a high analytical sensitivity and specificity for detection of bacterial DNA in blood.

Said *cfb* gene of *S. agalactiae* is a Christie Atkins Munch-Petersen (CAMP) factor gene of group B streptococci. CAMP factor b enhances hemolysis as a result of the production of a heat stable, filterable agent produced by only group B streptococci such as *S. agalactiae.* A coding sequence of the cfb gene of *S. agalactiae* strain ZQ0908 is provided in GenBank accession number HQ148672.1. Preferred primers and probes are indicated in Table 1.

Said *gyrB* gene of *Serratia marcescens* is a gene that encodes a DNA gyrase, a type II DNA topoisomerase, which is an enzyme capable of transforming relaxed closed circular DNA into a negatively supercoiled form. The enzyme contains two protein subunits, subunits A and B. The B subunit, encoded by *gyrB,* mediates energy transduction and ATP hydrolysis during the topological transformation of DNA. A partial sequence of this gene *of S. marcescens* is provided, for example, by GenBank accession number AJ300536.1. Preferred primers and probes are indicated in Table 1.

A preferred non-neonatal, preferably adult, sepsis test comprises detection of the *phzE* gene or *phzE* gene product, or a part thereof, of *P. aeruginosa,* the *rhaA* gene or *rhaA* gene product, or a part thereof, of *Klebsiella* spp, and detection of *Escherichia coli,* preferably *gadA and gadB* gene, *Acinetobacter baumannii,* preferably a 23S rRNA gene, *Enterococcus faecium,* preferably a gene encoding hypothetical protein, *Streptococcus pneumoniae,* preferably a *comX* gene, *Enterococcus* spp., preferably a 23S rRNA gene, Candida albicans, preferably a 18S-28S rRNA ITS region, *Candida glabrata,* preferably a 18S-28S rRNA ITS region, *Candida krusei,* preferably a 18S-28S rRNA ITS region, *Aspergillus* spp, preferably a 18S-28S rRNA ITS region, Gram positive bacteria, preferably a 16S rRNA gene, Gram negative bacteria, preferably a 16S rRNA gene, Candida spp. preferably a 18S-28S rRNA ITS region, and/or a gene or gene product selected from mecA, vanA, and ctxM, preferably ctxM-1 and/or ctxM-9. A most preferred non-neonatal sepsis test comprises detection of all indicated genes or regions.

Said 23S rRNA gene of *Acinetobacter baumannii* or of *Enterococcus* spp., is a gene that encodes a 23S ribosomal RNA (or 23S rRNA), which is a component of the 50S large subunit of prokaryotic ribosomes. The 23S rRNA gene is highly conserved between different species of bacteria. 23S rRNA gene sequences contain hypervariable regions that can provide species-specific signature sequences useful for bacterial identification. Annotated 23S ribosomal rRNA sequences are available at http://www.arb-silva.de. Preferred primers and probes for detection of a 23S rRNA gene of *Acinetobacter baumannii* or of *Enterococcus* spp., are indicated in Table 2.

Said hypothetical protein-encoding ORF gene of *Enterococcus faecium* is a hypothetical protein that is encoded by a gene with locus tag EFAU085_00469 that is, for example, located in a genomic region between nucleotides 489869 and 490153 of the genome of *Enterococcus faecium* Aus0085, as provided in GenBank accession number CP006620.1. Preferred primers and probes for detection of this ORF gene are indicated in Table 2.

Said *comX* gene of *Streptococcus pneumoniae* is an early response gene that is involved in a quorum-sensing system mediated by a peptide pheromone called competence-stimulating peptide (CSP), which acts to coordinate transient activation of genes required for competence. A coding sequence of the *comX* gene of *Streptococcus pneumoniae* is provided, for example, by GenBank accession number AF161701.1. Preferred primers and probes for detection of the *comX* gene are indicated in Table 2.

Said 18S-28S rRNA ITS region refers to the internal transcribed spacer region that is located in between the 18S and 5.8 S rRNA genes and/or between the 5.8 S and 28S rRNA genes. The ITS regions vary greatly in size and sequence in eukaryotic genomes, including fungi such as Candida spp. and Aspergillus spp. Said sequence preferably comprises the ITS2 region in between the 5.8 S rDNA gene sequence and the 28 S rDNA gene sequence of Aspergillus spp., or the ITS2 region in between the 5.8 S rDNA gene sequence and the 28 S rDNA gene sequence of Candida spp., including C. albicans, C. glabrata and C. krusei. Preferred primers and probes for detection of the 18S-28S rRNA ITS region in the indicated species are indicated in Table 2.

Said 16S rRNA gene of Gram positive and/or Gram negative bacteria is a gene that encodes a 16S ribosomal RNA (or 16S rRNA), which is a component of the 30S small subunit of prokaryotic ribosomes. The 16S rRNA gene is highly conserved between different species of bacteria. 16S rRNA gene sequences contain hypervariable regions that can provide species-specific signature sequences useful for bacterial identification. The Ribosomal Database Project (RDP) provides annotated ribosomal rRNA sequences along with related programs and services and is available at http://rdp.cme.msu.edu. Preferred primers and probes for detection of the Said 16S rRNA gene of Gram positive and/or Gram negative bacteria are indicated in Table 2.

Said *mecA* gene is a gene that confers resistance to antibiotics such as methicillin, penicillin and other penicillin-like antibiotics. In *Staphylococcus species, mecA* is spread on a SCCmec genetic element. Carriers of this genetic element are termed methicillin-resistant *S. aureus* or MRSA. A *mecA* coding sequence *of S. aureus* strain 13101 is provided in GenBank accession number JQ582126.1. Preferred primers and probes for detection of the *mecA* gene are indicated in Table 2.

Said *vanA* gene is a gene that confers resistance to vancomcin. In *Staphylococcus* species, *vanA* is encoded within a Tn1546 transposon located on a plasmid that is carried by vancomcin-resistant isolates. This transposon confers *vanA*-type vancomycin resistance in enterococci. A partial *vanA* coding sequence of *Enterococcus faecalis* strain VRE44 is provided in GenBank accession number JN207933.1. Preferred primers and probes for detection of the *vanA* gene are indicated in Table 2.

Said *ctxM* gene is a gene that encodes an extended spectrum β-lactamase. Coding sequence of three ctxM genes from three different *E. coli* isolates are provided in GenBank accession numbers EU935738, EU935739 and EU935740 (Woodford et al., 2009. Antimicrob Agents Chemother 53: 4472-4482). Preferred *ctxM* genes are *ctxM-1* and *ctxM-9* genes. A sequence comprising a *ctxM-1* gene is provided in GenBank accession number KJ802720.2. A sequence encoding a *ctxM-9* gene is provided in GenBank accession number JN676841.1. Preferred primers and probes for detection of the *ctxM-1* and *ctxM-9* genes are indicated in Table 2.

**Table 1. Primers and probes for neonatal multiplex real-time PCR**

| | **Species** | **PCR no** | **PCR target** | **Primer 1** | **Primer 2** | **Probe** | **Probe label 5'** | **Probe label 3'** |
|---|---|---|---|---|---|---|---|---|
| 1 | Escherichia coli | 2 | 16S rDNA | CATGCCGCGTGT ATGAAGAA | CGGGTAACGTCA ATGAGCAAA | TATTAACTTTACTCCCT TCCTCCCCGCTGAA | Atto425 | BHQ1 |
| 2 | Pseudomonas aeruginosa | 2 | phzE gene | GCCGAGGTCATG GAATTC | ATCCGCGCCATC ATCTTC | CGACAACCGCAAGGAAG CCGA | YakimaY ellow | BHQ1 |
| 3 | Klebsiella species | 1 | rhaA gene | AACCAGGCGTCG ATAAT | GTTTACGGCGCA ATCC | ACAGGAAAGACAAGACT ATGCAGACC | YakimaY ellow | BHQ1 |
| 4 | Serratia marcescens | 2 | gyrB gene | GACCGTGAAGAC CACTTCCATTAC | ACGCCGATGTCG TCTTTCAC | CGATCCACCCGAACGTG TTCTACTTCTC | ROX | BHQ2 |
| 5 | Staphylococcus species | 3 | tuf gene | CCAACTCCAGAA CGTGATTCTG | GTTGTCACCAGC TTCAGCGTAGT | ACAGGCCGTGTTGAACG TGGKCAAATCAA | Atto425 | BHQ1 |
| | | | | CCAACTCCAGAA CGTGACTCTG | GTTATCACCAGC TTCAGCGTAAT | | | |
| | | | | CCAACACCAGAA CGTGATTCTG- | GTTGTCACCAGC TTCAGCATAGT | | | |
| 6 | Staphylococcus aureus | 1 | SA442 | CATCGGAAACAT TGTGTTCTGTAT G | TTTGGCTGGAAA ATATAACTCTCG TA | AAGCCGTCTTGATAATC TTTAGTAGTACCGAAGC TGGT | Atto425 | BHQ1 |
| 7 | Enterococcus faecalis | 1 | 16S rDNA | CGCTTCTTTCCT | GCCATGCGGCAT AAACTG | CAATTGGAAAGAGGAGT GGCGGACG | FAM | BHQ1 |
| 8 | Streptococcus agalactiae | 1 | cfb gene | TTCACCAGCTGT ATTAGAAGTACA TGC | CCCTGAACATTA TCTTTGATATTT CTCA | CAAGCCCAGCAAATGGC TCAAAAGCT | ROX | BHQ2 |

**Table 2. Primers and probes for non-neonatal multiplex real-time PCR**

| | **Species** | **PCR no** | **PCR target** | **Primer 1** | **Primer 2** | **Probe** | **Probe label 5'** | **Probe label 3'** |
|---|---|---|---|---|---|---|---|---|
| 1 | Escherichia coli | 2 | gadA + gadB | GGCTTCGAAATG GACTTTGCT | TGGGCAATACCC TGCAGTTT | CTGTTGCTGGAAGA CTACAAAGCCTCCC TG | Atto425 | BHQ1 |
| 2 | Acinetobacter baumannii | 2 | 23S rDNA | CGCTGTTGTTGG TGATGGAACT | AACAGTTGCAGC GGCCTG | CTTCCTGAGCTGAC GACAGCCGC | HEX | BHQ1 |
| 3 | Enterococcus faecium | 2 | hyp. protein encoding ORF | GCCAAAGGACCG CTTATTACG | GCTTTTCGCTGT TTTTTTAATGAC T | TTCGCAAGCGACAA CAAGCACAAGC | FAM | BHQ1 |
| 4 | Staphylococcu s aureus | 3 | hsdM gene | AAGGCGGAGGAA TCACATGTC | TTCGCAATCGAC CATAATTTTTT | TTACTGAAAAACAA CGTCAGCA | Atto425 | BHQ1 |
| 5 | Enterococcus faecalis | 3 | ncRNA | ATGCGTCTCGTC ACAGTA | GGTACGATGATT TCATCTGT | AGTTGCGATGTTTC ACTGTGAAGCA | FAM | BHQ1 |
| 6 | Pseudomonas aeruginosa | 3 | phzE gene | GCCGAGGTCATG GAATTC | ATCCGCGCCATC ATCTTC | CGACAACCGCAAGG AAGCCGA | HEX | BHQ1 |
| 7 | Streptococcus pneumoniae | 3 | comX gene | GGTCTCTGGCTA GATGATTATTAT CTCTT | ATAGTAAACTCC TTAAACACAATG CGTAA | CGCCCTCGAAATCG TTCATTGCTTAAGA | Cal635 | BHQ2 |
| 8 | Staphylococcu s species | 4 | tuf gene | CCAACTCCAGAA CGTGATTCTG | GTTGTCACCAGC TTCAGCGTAGT | ACAGGCCGTGTTGA ACGTGGKCAAATCA A | Atto425 | BHQ1 |
| | | | | CCAACTCCAGAA CGTGACTCTG | GTTATCACCAGC TTCAGCGTAAT | | | |
| | | | | CCAACACCAGAA CGTGATTCTG- | GTTGTCACCAGC TTCAGCATAGT | | | |
| 9 | Klebsiella species | 4 | rhaA gene | AACCAGGCGTCG ATAAT | GTTTACGGCGCA ATCC | ACAGGAAAGACAAG ACTATGCAGACC | HEX | BHQ1 |
| 10 | Enterococcus genus | 4 | 23S rDNA | TGCGGGATGAG GTGTG | CAAACAGTGCTC TACCTCCATCAT | TAGCCCTAAAGCTA TTTCGGAGAGAACC A | FAM | BHQ1 |
| 11 | Candida albicans | 4 | ITS region | CATGCCTGTTTG AGCGTCRTTT | ATATGCTTAAGT TCAGCGGGT | TAAGGCGGGATCGC TTTGACA | Ca1610 | BHQ2 |
| 12 | Candida glabrata | 1 | ITS region | CATGCCTGTTTG AGCGTCRTTT | ATATGCTTAAGT TCAGCGGGT | ATCAGTATGTGGA [?] CACGAGCG | Cal635 | BHQ2 |
| 13 | Candida krusei | 3 | ITS region | CATGCCTGTTTG AGCGTCRTTT | ATATGCTTAAGT TCAGCGGGT | ACGACGTGTAAAGA GCGTCGG | Ca1610 | BHQ2 |
| 14 | Pan-Aspergillus | 1 | ITS region | GCGTCATTGCTG CCCTCAAGC | ATATGCTTAAGT TCAGCGGGT | CCTCGAGCGTATGG GGC | Atto425 | BHQ1 |
| 15 | Gram-positive | 1 | 16S rDNA | TGGAGCATGTGG TTTAATTCGA | TGCGGGACTTAA CCCAACA | TGGTGCATGGTTG | FAM | BHQ1 |
| 16 | Gram - negative | 1 | 16S rDNA | TGGAGCATGTGG TTTAATTCGA | TGCGGGACTTAA CCCAACA | TGCTGCATGGCTGT | HEX | BHQ1 |
| 17 | pan-Candida | 1 | ITS region | CATGCCTGTTTG AGCGTCRTTT | ATATGCTTAAGT TCAGCGGGT | TCGTATTGCTCAAC ACCAAACCC | Ca1610 | BHQ2 |
| 18 | MecA | 2 | mecA gene | GATCGCAACGTT CAATTTAATTTT | GCTTTGGTCTTT CTGCATTCCT | AATGACGCTATGAT CCCAATCTAACTTC CACAT | Ca1610 | BHQ2 |
| 19 | VanA | 5 | vanA gene | CGGTTTCACGTC ATACAGTCGTT | CAGTTCGGGAAG TGCAATACC | TCCCCGTATGATGG CCGCTGC | HEX | BHQ1 |
| 20 a | CTXM | 5 | ctxm-1 gene | ATGTGCAGYACC AGTAARGTKATG GC | ATCACKCGGRTC GCCNGGRAT | CCCGACAGCTGGGA GACGAAACGT | FAM | BHQ1 |
| 20 b | CTXM | 5 | ctxm-9 gene | ATGTGCAGYACC AGTAARGTKATG GC | ATCACKCGGRTC GCCNGGRAT | CTGGATCGCACTGA ACCTACGCTGA | FAM | BHQ1 |

Inhibition of PCR can result in false negative results, and can seriously hamper the identification of specific micro-organisms in a sample. Inhibition can be assessed by using an internal control and primers that are specific for that internal control. If the internal control is not amplified to its normal levels, it can be deduced that the PCR reaction was inhibited.

The *gB* gene of Phocine herpesvirus 1 (PhHV-1) was used as internal control in multiplex amplification assays, as described in van Doornum et al. 2003 (van Doornum et al. 2003. J Clin Microbiol 41: 576-80). Said internal control is preferably added as virus to a blood sample prior to or during the step of isolating DNA. The amount of an internal control that is added to a real time PCR reaction is preferably such that amplification of the internal control does not influence the pathogen-specific amplification reactions. The amount is preferably such that the resulting Ct value of the internal control is about 37 cycli.

A preferred forward primer for the *gB* gene comprises the sequence 5'-GGGCGAATCACAGATTGAATC-3', a preferred reverse primer comprises the sequence 5'-GCGGTTCCAAACGTACCAA-3', and a preferred probe comprises the sequence 5'-TTTTTATGTGTCCGCCACCATCTGGATC-3'. Said probe is preferably labelled with Atto647N. The amplified fragment using the preferred primers is 89 bp.

Multiplex real-time PCR employs several primer sets and probes in the same reaction tube. The presence of multiple primers may lead to cross hybridization with each other and the possibility of mis-priming with other templates, which is to be avoided. The individual amplification products in a multiplex reaction may differ in size by at least 15 bp to allow sufficient fragment length resolution for size discrimination by gel electrophoresis.

It is preferred to design the internal control (IC) probe for detection of the gB gene of PhHV-1 to emit in the channel with the highest wavelength, as fluorescence is usually lower at higher wavelengths. This ensures that the IC assay will suffer most from any inhibition of the amplification reaction. It is further preferred to place the assay for detection of *Staphylococcus* species in a separate amplification reaction as the sensitivity of this assay was reduced in multiplexed format.

As an alternative, or in addition, to the internal control, internal amplification controls (IAC) may be used for each multiplex. IAC are constructed such that a forward primer from one reaction and a reverse primer from another reaction will produce an amplified region comprising an artificial sequence. This artificial sequence, or a part thereof, preferably serves as a probe sequence. Said amplified region preferably is greater in length than the amplified regions of the indicated micro-organisms. The reason for this is that the IAC preferably does not impact the pathogen-specific detection amplification reactions. A greater length of the amplified region of the IAC will result in an optimal amplification of the pathogen-specific detection amplification reactions, while amplification of the IAC may be less optimal when more micro-organisms are present in the blood sample that are detected by use of the forward primer and/or the reverse primer.

The amplified region of an IAC is preferably cloned into a vector. It is preferred that said vector comprises an amplified region comprising an artificial sequence that is amplified by at least two different primer pairs. Such IAC may than be used in separate multiplex PCR assays using the same labelled probe. For example, an *Escherichia coli* reverse primer for the *gadA*/*gadB* gene having the sequence 5'-TGGGCAATACCCTGCAGTTT (See Table 2) may be used together with a *A*. *baumanii* forward primer 5'-CGCTGTTGTTGGTGATGGAACT (See Table 2) to amplify an artificial sequence comprising the nucleotide sequence 5'-TCTGGCGAAAGATTTGGCGGATGTGCATT. A probe comprising the sequence 5'-TCTGGCGAAAGATTTGGCGGATGTGCATT that is labelled at its 5' end with Atto647N may be used as a probe for detection of the internal amplification control. It is preferred that 10-1000, preferably 100-200, preferably about 150 copies of an IAC, preferably as a plasmid, are added to a sample prior to a multiplex PCR reaction. An IAC probe is preferably added at 50-500 nM, more preferred at about 200 nM, per PCR reaction.

If required, a specific blocker oligonucleotide might be added to an amplification reaction that prevents hybridization of a primer to a specific conflicting target sequence, for example a target sequence on a related micro-organism. The blocker oligonucleotide does not prevent the hybridization of the primer to the specific target DNA molecule or the internal amplification control. A blocker oligonucleotide is preferably used when there is a highly taxonomically related species that preferably should not function as a target DNA molecule or when a specific sequence negatively influence the amplification of a specific target DNA molecule.

A preferred blocker oligonucleotide comprises one or more modifications that prevent its use as an amplification primer. For example, the 3'-end of a blocker oligonucleotide may be phosphorylated, thereby preventing DNA elongation. Other modifications, including but not limited to the incorporation of modified nucleotides, such as LNAs, or an increased size as compared to the primer and/or probe, are possible, DNA elongation from the blocking oligonucleotide is hampered or prevented, and hybridization of the primer to a specific conflicting target sequence is prevented. This will result in a normal exponential amplification of the specific target DNA molecule.

The indicated sepsis tests may be performed as individual single template PCR assays, also termed monoplex assays. Most real-time PCR platforms are able to detect fluorescence at at least 6 different emission wavelength windows (channels). Therefore, it is preferred to combine a maximum of 6 single template PCR reactions in one multiplex PCR assay. This will reduce the amounts of reagents including the amount of target nucleic acid template and preparation time, compared to individual single template PCR reactions.

Each multiplex PCR assay preferably includes at least one internal control, preferably the *gB* gene of PhHV-1 as is indicated herein above. Further control reactions include at least one negative control, internal amplification controls and quantitative controls, as defined herein, which are preferably run in separate multiplex PCR assays that are preferably set up and run in parallel to the pathogen detecting multiplex PCR assays using the same batches of primers, probes, enzymes and buffer systems.

It is additionally preferred to design probes detecting the most prevalent bacteria (*E. coli, S. aureus, Staphylococcus* spp.) to emit in the lowest wavelength channels. This positions them in separate multiplex PCR assays.

Typical reaction conditions for use on a LightCycler 480II instrument included 12.5 µl of 2x LightCycler 480 Probes Master mix (Roche Diagnostics, Almere, The Netherlands), 2.5 µl primers and probe(s) and 10 µl DNA input, resulting in a final reaction volume of 25 µl. It is additionally preferred to use primers at a concentration between 100 and 1200 nM, preferably between 300 nM to 900 nM, more preferred about 900 nM, and to use probes at a concentration between 100 and 500 nM, preferably between 150 nM and 200 nM, since this was found to result in high fluorescence.

The LightCycler Master mix may be replaced by the 2x QuantiFast Multiplex PCR Master Mix (Qiagen, Venlo, The Netherlands) to reduce background signals. Routine cycling conditions were 10 min at 95°C followed by 45 cycles of 95°C for 15 sec and 60°C for 1 min.

As is known to a person skilled in the art, color compensation may be applied to correct for fluorescent bleed-through.

### 5.4 Primers and probes

The invention additional provides at least one forward or reverse primer or probe as defined herein, preferably wherein at least one of said at least one forward or reverse primer or probe comprises a detectable label, preferably a fluorescent label. The forward or reverse primer or probe as defined herein are preferably used for detection of at least one microorganism as is described herein, preferably in a method for detecting sepsis according to the present invention.

Said at least one forward or reverse primer or probe preferably comprises a set of primers, comprising a forward and reverse primer that can be used to amplify a region on a target DNA molecule. The set of primers preferably further includes a probe, preferably a fluorescently labelled probe, that recognizes the amplified region on the target DNA molecule.

It is preferred that the probe is labeled, preferably at the 5' end, with a fluorescent label, preferably selected from Atto425, YakimaYellow, ROX, Cal610, Cal635, FAM, TET, HEX, Cy5, Cy5.5, Cy3, Cy3.5, Cy7, Alexa dyes Tamra, ROX, JOE, FITC, and TRITC.

The invention further provides a kit of parts adapted for performing a method of the invention, said kit comprising at least one forward or reverse primer or probe as defined herein, preferably wherein at least one of said at least one forward or reverse primer or probe comprises a detectable label, preferably a fluorescent label.

A preferred kit comprises a forward and reverse primer that can be used to amplify a region on a target DNA molecule. The kit preferably further includes a probe, preferably a fluorescently labelled probe, that recognizes the amplified region on the target DNA molecule.

A preferred kit comprises at least primers and probe for amplification of the *phzE* gene or *phzE* gene product, or a part thereof, of *Pseudomonas aeruginosa,* preferably wherein a forward primer comprises the sequence 5'-GCCGAGGTCATGGAATTC-3', a reverse primer comprises the sequence 5'-ATCCGCGCCATCATCTTC-3', and a probe comprises the sequence 5'-CGACAACCGCAAGGAAGCCGA-3'.

A preferred kit comprises at least primers and probe for amplification of the *rhaA* gene or *rhaA* gene product, or a part thereof, of *Klebsiella* spp., preferably wherein a forward primer comprises the sequence 5'-AACCAGGCGTCGATAAT-3', a reverse primer comprises the sequence 5'-GTTTACGGCGCAATCC-3', and a probe comprises the sequence 5'-ACAGGAAAGACAAGACTATGCAGACC-3'.

A further preferred kit, especially for determining sepsis in neonatals, comprises at least primers and probe for amplification of the *phzE* gene or *phzE* gene product, or a part thereof, of *Pseudomonas aeruginosa,* primers and probe for amplification of the *rhaA* gene or *rhaA* gene product, or a part thereof, of *Klebsiella* spp., and primers and probe for detection of at least one microorganism selected from the group consisting of *Enterococcus faecalis, Escherichia coli, Staphylococcus* spp. *Streptococcus agalactiae, Serratia marcescens* and *Staphylococcus aureus.*

A preferred neonatal sepsis kit preferably includes instructions for setting up three multiplex amplification reactions, wherein a first amplification reaction comprises the amplification of a gene or gene product, or a part thereof, of *Staphylococcus aureus, Enterococcus faecalis, Klebsiella* spp., and *Streptococcus agalactiae*; a second amplification reaction comprises the amplification of a gene or gene product, or a part thereof, of *Escherichia coli, Pseudomonas aeruginosa,* and *Serratia marcescens*; and wherein a third amplification reaction comprises the amplification of a gene or gene product, or a part thereof, of *Staphylococcus* spp.

A further preferred kit, especially for determining sepsis in non-neonatals, preferably adults, comprises at least primers and probe for amplification of the *phzE* gene or *phzE* gene product, or a part thereof, of *Pseudomonas aeruginosa,* primers and probe for amplification of the *rhaA* gene or *rhaA* gene product, or a part thereof, of *Klebsiella* spp., and primers and probe for detection of at least one microorganism selected from the group consisting of *Enterococcus faecalis, Escherichia coli, Staphylococcus* spp., *Staphylococcus aureus,* and primers and probe for detection of at least one microorganism selected from the group consisting of *Acinetobacter baumannii, Enterococcus faecium, Streptococcus pneumoniae, Enterococcus spp., Candida* spp., *Candida albicans, Candida glabrata, Candida krusei, Aspergillus* spp, Gram positive bacteria, Gram negative bacteria, or a gene or gene product selected from mecA, vanA, and ctxM.

A preferred non-neonatal sepsis detection kit preferably comprises primers and probe for amplification of the *phzE* gene or *phzE* gene product, or a part thereof, of *Pseudomonas aeruginosa,* for amplification of the *rhaA* gene or *rhaA* gene product, or a part thereof, of *Klebsiella* spp., for detection of *Enterococcus faecalis, Escherichia coli, Staphylococcus* spp., *Staphylococcus aureus, Acinetobacter baumannii, Enterococcus faecium, Streptococcus pneumoniae, Enterococcus spp., Candida* spp., *Candida albicans, Candida glabrata, Candida krusei, Aspergillus* spp, Gram positive bacteria, Gram negative bacteria, and mecA, vanA, and ctxM.

Said preferred non-neonatal sepsis detection kit includes instructions for setting up five multiplex amplification reactions, wherein a first amplification reaction comprises the amplification of a gene or gene product, or a part thereof, of *Aspergillus* spp, Gram positive bacteria, Gram negative bacteria, *Candida* spp. and *Candida glabrata*; a second amplification reaction comprises the amplification of a gene or gene product, or a part thereof, of *Escherichia coli, Enterococcus faecium, Acinetobacter baumannii,* and the *mecA* gene; a third amplification reaction comprises the amplification of a gene or gene product, or a part thereof, of *Staphylococcus aureus, Enterococcus faecalis, Pseudomonas aeruginosa, Candida krusei,* and *Streptococcus pneumoniae;* a fourth amplification reaction comprises the amplification of a gene or gene product, or a part thereof, of *Staphylococcus* spp., *Enterococcus* spp., *Klebsiella* spp. and *Candida albicans,* and a fifth amplification reaction comprises the amplification of a gene or gene product, or a part thereof, of *vanA* and *ctxM.*

Control reactions, preferably including at least one negative control, an internal control and internal amplification controls are preferably included in a preferred neonatal and/or non-neonatal sepsis detection kit.

### 5.5 Treatment

The methods and kits for diagnosing sepsis in mammals are preferably succeeded by prompt and effective treatment of the active infection, which is essential to the successful treatment of severe sepsis and septic shock. Intravenous antibiotic therapy should be initiated as soon as possible, preferably within the first hours after obtaining results from a sepsis test according to this invention, since early initiation of antibiotic therapy is associated with lower mortality. The choice of antibiotics can be complex and should consider the patient's history, for example whether the patient recent received antibiotics, comorbidities, and clinical context (for example, community- or hospital-acquired).

It is an aspect of this invention to provide products and methods for determining the presence of microbial contaminants in blood (i.e. microbial blood infections), preferably in a near-patient situation, whereby the results of the methods for determining said presence are preferably available within 4 hrs, preferably within 3 hrs, more preferably within 2 hrs, even more preferably within 1 hr from the moment that a blood sample from a patient is provided. Such a near-patient test represents a system for assisting a physician to assign to a human patient an appropriate antimicrobial therapy.

This will reduce the occurrence of inadequate antimicrobial treatment, i.e. infections that are not being effectively treated. The emergence of antibioticresistant bacteria is for an important part due to inadequate antimicrobial treatment, which is itself due, in part, to the use of broad specterum antibiotics

In order to reduce the risk of inadequate antimicrobial treatment, it is preferred that a system according to the present invention is used, wherein said system comprises means for performing a method for determining the presence of microbial contaminants in blood as described herein above. In highly preferred embodiments, the method comprises the step of performing on a blood sample from a subject suspected of suffering from a microbial contaminant in blood, or on a sample of nucleic acids isolated therefrom, a nucleic acid amplification reaction, wherein said nucleic acid amplification reaction comprises the amplification of a gene or gene product, or a part thereof, of at least the following targets:
- Gram-positive bacteria,
- Gram-negative bacteria,
- at least one genus of fungi, and
- at least one antibiotic resistance gene, preferably of a multidrug-resistant microorganism.

The terms "Gram-positive" and "Gram-negative" bacteria as used in this aspect of the invention, and, unless otherwise indicated, also in other aspects of this invention, should be understood herein as referring to a class of bacteria that do or do not, respectively, retain the crystal violet stain used in the Gram staining method of bacterial differentiation. The two different bacterial classes can be detected individually using the class-specific PCR detection methods as described herein.

The term "fungi" as used in this aspect of the invention, and, unless otherwise indicated, also in other aspects of this invention, should be understood herein as referring to a group of eukaryotic organisms that includes yeasts, molds and mushrooms. These organisms are classified as a kingdom, Fungi. The at least one genus of fungi can be detected at any suitable taxonomic level higher than the individual species level, including the level of multiple species of said genus using genus-level PCR detection methods. Preferably, a method of the invention comprises the detection of at least one genus of fungi. Alternatively, 2, 3, 4, or more genera of fungi may be detected. The term genus is meant to refer to multiple microbial species of a single taxonomic genus, such as 2, 3, 4, 5, 6, or more species belonging to a single genus, including all species of a genus. Since genus-level detection is sometimes hampered by the absence of common primer-binding sequences in the target amplification region in some species of a genus, primers and probes for genus level detection may generally include ambiguous bases to facilitate hybridization to species having a number of divergent bases in the primer and/or probe target sequence. A set of amplification primers and detection probe(s) for genus-level detection specifically amplifies the target sequence from at least 2, preferably 3, 4, 5 or more, preferably all, species of said genus. Genus-level detection, including detection of multiple species of a single genus (indicated herein by the term "<genus name> spp."), or even multiple-genera, such as family level detection, the contents of which should be understood as being incorporated in the term genus-level detection, is advantageous in that only a single PCR amplification reaction needs to be performed the establish the presence or absence of a large number of microorganisms simultaneously. Thus, this system of genus detection in methods of this invention is very suitable also for detection of certain genera of bacterial blood contaminant as indicated herein. In order to determine the presence, or absence, of a multitude of species as part of the genus-level detection format described herein, preferably at least those microbial species that are known blood contaminants or sepsis-causing organisms are included in the genus-specific detection format. Preferred embodiments of a method of the invention as described herein for detection of at least one fungal genus comprise the genus-level detection of *Candida* spp. or *Aspergillus* spp., preferably *Candida* spp. and *Aspergillus* spp. using the genus-level PCR detection methods as described herein. In preferred embodiments, genus level is genus-specific.

The term "antibiotic resistance gene", refers to a gene that confers antibiotic resistance to a micro-organism. The gene may encode an enzyme which degrades or excretes an antibiotic, or that prevents antibiotics from entering the cell, thereby conferring resistance. Preferably the antibiotic resistance gene is a gene from a multidrug-resistant microorganism.

The detection system for detecting microbial blood infections of this invention in preferred embodiments outlined above has the advantage of having very broad species-coverage, while still providing information on the taxonomic identity of the contaminant that is useful for immediate commencement of antimicrobial therapy, without having to resort to a broad-spectrum antibiotic. Preferably, in aspects of the invention, the method for determining the presence of microbial contaminants in blood therefore further comprises the provision of listing of a number of candidate antimicrobials, not being broad-spectrum antibiotics, that may stop the propagation of the microbial contaminant that is detected in the blood.

In preferred embodiments of a method of detecting microbial infections in blood, the method further comprises the detection of a number of species-specific microbial detection tests including *Pseudomonas aeruginosa, Klebsiella species, Escherichia coli, Acinetobacter baumannii, Enterococcus faecalis, Enterococcus faecium, Staphylococcus species, Staphylococcus aureus, Streptococcus pneumoniae, Streptococcus agalactiae, Serratia marcescens, Candida albicans, Candida glabrata,* and *Candida krusei,* as described herein above. Very suitable, the species-specific detection in methods of this invention includes the specific detection of some 5, 7, 10, 12, 15, or 20 species of micro-organisms, including, preferably, known microbial blood contaminants, preferably including known sepsis-causing microorganisms. It is an advantage of the present invention of including a detection of microbial species at a higher-taxonomic level of detection as provided by the Gram-positive, Gram-negative, and Fungal genus detection, that the failure to detect a specific species of a micro-organisms does not result in a negative test. The microbial contaminant in the patient's blood may still be detected at a higher taxonomic level of identification as indicated above, thereby preventing false negative results. At the same time, even though no specific species identification of the microbial contaminant could be made in the case the contaminant is not covered by the species-specific sets of primers provided, the result of the test performed in accordance with a method of this invention still allows the commencement of an appropriate antimicrobial therapy, without having to use broad-spectrum antibiotics.

Hence, it is an aspect of the invention to provide a method of treating a subject suffering from microbial blood contaminants, wherein said method, in addition to the microbial detection methods as outlined above, comprises the administration of an antibiotic to a subject in need thereof, said antibiotic preferably not being a broad-spectrum antibiotic. The term "broad spectrum antibiotic", as used herein, refers to an antimicrobial agent that is effective against both gram-positive and gram-negative bacteria.

Aspects of the methods of the present invention wherein treatment is performed following the specific detection formats as outlined herein include embodiments wherein the treatment is started within 4 hrs, preferably within 3 hrs, still more preferably within 2 hrs after blood retreival.
In contrast to methods of the prior art wherein species-specific detection formats are taught for diagnosis of sepsis, and wherein the aim is to determine the exact taxonomic position of the microbial blood contaminant in order to then determine the most suitable antimicrobial treatment scheme, it is an aim and advantage of the methods of this invention that the exact taxonomic position of the microorganism does not need to be precisely determined, but that an effective antimicrobial treatment strategy, not including the use of broad spectrum antibiotics but comprising the administration of one or more selected narrow-spectrum antimicrobials, can be ascertained from the test results within a very short timespan and with a high level of certainty. The narrow-spectrum antibiotic is active against a selected group of microbial types or groups including, for instance, Gram-negative bacteria, Gram-positive bacteria, fungi, or multi-resistant bacterial strains.

The invention further provides a method of treating a subject suffering or suspected of suffering from sepsis, the method comprising determining an amount of amplified nucleic acid with a method according to the invention and treating said subject with a specific antibiotic if one or more of *Pseudomonas aeruginosa, Klebsiella species, Escherichia coli, Acinetobacter baumannii, Enterococcus faecalis, Enterococcus faecium, Staphylococcus species, Staphylococcus aureus, Streptococcus pneumoniae, Streptococcus agalactiae, Serratia marcescens, Candida albicans, Candida glabrata, Candida krusei, Pan-Aspergillus,* pan-*Candida,* Gram-positive bacteria, Gram-negative bacteria, MecA, VanA, and/or CTXM, is detected.

If *Pseudomonas aeruginosa* is found to be a cause of sepsis with a method of the invention, treatment preferably includes a beta-lactam/beta-lactamase inhibitor such as imipenem for a neonatal subject. A non-neonatal subject is preferably treated with a cephalosporin such as ceftazidime and/or with an extended-spectrum beta-lactam antibiotic of the ureidopenicillin class such as, for example, piperacillin. Said extended-spectrum beta-lactam antibiotic is preferably combined with a beta-lactamase inhibitor, preferably tazobactam.

If *Klebsiella* spp. is found to be a cause of sepsis with a method of the invention, treatment preferably includes a beta-lactam/beta-lactamase inhibitor such as imipenem for a neonatal subject. A non-neonatal subject is preferably treated with a cephalosporin such as ceftriaxon.

A neonatal subject is preferably treated with imipenem if said subject is suffering from sepsis that is caused by *E. coli, Acinetobacter baumanii,* and/or *Serratia marcescens,* as determined with a method of the invention.

A neonatal subject is preferably treated with a narrow spectrum beta-lactam antibiotic such as flucloxacillin if said subject is suffering from sepsis that is caused by *S. aureus,* as determined with a method of the invention; with a moderate-spectrum, beta-lactam antibiotic such as amoxicillin if said subject is suffering from sepsis that is caused by *Enterococcus faecalis,* as determined with a method of the invention; with a glycopeptide antibiotic such as vancomycin if said subject is suffering from sepsis that is caused by *Staphylococcus* spp. or by *E. faecium,* as determined with a method of the invention; with a lipopeptidic antifungal drug such as caspofungin if said subject is suffering from sepsis that is caused by *Candida* species; with a glycopeptide antibiotic such as vancomycin if said subject is suffering from sepsis that is caused by *E. faecium,* as determined with a method of the invention; with a beta-lactam antibiotic such as penicillin if said subject is suffering from sepsis that is caused by *Streptococcus agalactiae* or *S. pneumonia,* as determined with a method of the invention;

A non-neonatal subject, preferably an adult, is preferably treated with a narrow spectrum beta-lactam antibiotic such as flucloxacillin if said subject is suffering from sepsis that is caused by *S. aureus,* as determined with a method of the invention; with a cephalosporin such as ceftriaxon if said subject is suffering from sepsis that is caused by *E. coli;* with a moderate-spectrum, beta-lactam antibiotic such as amoxicillin if said subject is suffering from sepsis that is caused by *Enterococcus faecalis,* as determined with a method of the invention; with a cephalosporin such as ceftazidime if said subject is suffering from sepsis that is caused by A. *baumanii,* as determined with a method of the invention; with a glycopeptide antibiotic such as vancomycin if said subject is suffering from sepsis that is caused by *E. faecium,* as determined with a method of the invention; with a semisynthetic echinocandin such as anidulafungin if said subject is suffering from sepsis that is caused by *Candida* species, as determined with a method of the invention; with a broad-spectrum quinoline such as ciprofloxacin or with a combination of antifolate substances such as trimethoprim/sulfamethoxazole (co-trimoxazole) if said subject is suffering from sepsis that is caused by *Serratia marcescens,* as determined with a method of the invention; with a glycopeptide antibiotic such as vancomycin if said subject is suffering from sepsis that is caused by *Staphylococcus* spp., as determined with a method of the invention; with a beta-lactam antibiotic such as penicillin if said subject is suffering from sepsis that is caused by *Streptococcus agalactiae* or *S. pneumonia,* as determined with a method of the invention.

Therefore, the invention also provides a method of treating a subject suffering or suspected of suffering from sepsis, the method comprising determining an amount of amplified nucleic acid with a method for detecting sepsis according to the invention, and providing an antimicrobial compound, preferably an antimicrobial compound as is indicated herein above, if the if said subject is suffering from sepsis that is caused by one of the specified microorganisms.

The methods of the invention provide a fast diagnostic tool for monitoring the course of sepsis. Said methods are very well suited to monitor the course of anti-sepsis treatment by taking blood samples from a patient at different time points, for example every 4 hours, every 8 hours, every 12 hours or, preferably every 24 hours after start of a treatment with an antibiotic, and quantitatively determining the amount of one or more of the indicated micro-organisms in the sample. A decrease in the amount of said one or more of the indicated micro-organisms over time, for example within 2-6 days, preferably within 2-4 days, is indicative of a successful treatment resulting in clearance of the infection. No decrease in the amount of said one or more of the indicated micro-organisms over time is indicative of a persistent infection. The finding of a persistent infection is an indication that treatment may be altered, for example by addition of a second antibiotic, or by selection of another antibiotic.

Systemic inflammatory response syndrome (SIRS) is clinical syndrome that resembles sepsis. However, SIRS is not associated with an infection but complicates a noninfectious insult such as acute pancreatitis or pulmonary contusion. It is thought that dysregulation of the inflammatory response, including an uncontrolled release of pro-inflammatory mediators, initiates a chain of events that lead to widespread tissue injury. This response can lead to multiple organ dysfunction syndrome (MODS), which is the cause of high mortality associated with both sepsis and SIRS.

The invention provides a method for differentiating between patients suffering from sepsis and patients suffering from systemic inflammatory response syndrome (SIRS), said method comprising performing a method according to the invention. The identification of a microorganism selected from *Pseudomonas aeruginosa, Klebsiella* spp., *Enterococcus faecalis, Escherichia coli, Staphylococcus* spp., *Staphylococcus aureus, Acinetobacter baumannii, Enterococcus faecium, Streptococcus pneumoniae, Enterococcus spp., Streptococcus agalactiae, Serratia marcescens, Candida* spp., *Candida albicans, Candida glabrata, Candida krusei, Aspergillus* spp, Gram positive bacteria, or Gram negative bacteria indicates that the patient is suffering from sepsis.

### Examples

### Example 1

### Materials and methods

### Bacterial strains

A panel of 38 bacterial and 3 fungal species that are either known to cause neonatal sepsis or are regular contaminants of blood culture was selected (Table 3). Strains were obtained from the American Type Culture Collection (ATCC), Deutsche Sammlung von Mikroorganismen und Zellkulturen (DSMZ), Center for Disease Control (CDC) and the culture collections of the microbiology laboratories of the VU University Medical Center (Amsterdam, The Netherlands), University Medical Center Utrecht (Utrecht, The Netherlands) and Radboud University Nijmegen Medical Centre (Nijmegen, The Netherlands). Clinical strains were identified using standard phenotypic and genotypic laboratory procedures, 16S rDNA sequencing (Nijmegen), Phoenix Automated Microbiology System (Utrecht) and MALDI111 TOF VitekMS (bioMérieux) or amplified fragment length polymorphism (AFLP) (Amsterdam).

### DNA isolation from pure cultures and blood

To obtain microbial DNA from pure cultures, microorganisms from fresh plate cultures were suspended into tryptic soy culture broth (enriched with 10% horse serum and 6 µgram/ml nicotinamide adenine dinucleotide) and grown at 37°C to an optical density (OD) of 0.5 McFarland or directly suspended in phosphate-buffered saline (PBS) to an OD of 0.5 McFarland. Cells were collected from 200 µl of the 0.5 McFarland suspension after centrifugation (10 min at 14,000x g) and subsequently lysed by incubation for 10 minutes at 95°C whilst shaking (800 rpm) in 200 µl of bacterial lysis buffer (BLB) (Biocartis, Mechelen, Belgium). After addition of 20 µl neutralization buffer (Biocartis, Mechelen, Belgium), microbial DNA was extracted with the QIAamp DNA mini kit (Qiagen, Venlo, The Netherlands). DNA quantity is given as colony forming unit equivalents (cfu eq), e.g.10 cfu eq represents DNA isolated from 10 cfu. Measurement of OD and bacterial plate counts were used to determine cfu.

For isolation of microbial DNA from blood, 200 µl of EDTA anticoagulated-blood was treated twice with 1 ml of TTE (1% Triton X-100, 20 mM Tris-HCl pH 8.3 and 1 mM EDTA) to lyse erythrocytes and remove hemoglobin as described (Peters et al. 2007. J Clin Microbiol 45: 3641-6). The resulting bacterial pellet was lysed with BLB as described earlier. Subsequently, DNA was purified with the NucliSENSEasyMAG device (bioMérieux, Zaltbommel, The Netherlands) using protocol "Specific A" in an elution volume of 110 µl. The manufacturer's instructions were followed except that 1 ml AL buffer (Qiagen, Venlo, The Netherlands) plus 1 ml easyMAG Lysis buffer (bioMérieux, Zaltbommel, The Netherlands) were used instead of 2 ml easyMAG Lysis buffer during DNA binding to the beads. The AL buffer was spiked with Phocine Herpesvirus 1 (PhHV-1) as extraction and amplification control (internal control) (van Doornum et al. 2003. J Clin Microbiol 41: 576-80). Successful bacterial lysis and subsequent DNA extraction was verified with a broad-range or pathogen-specific PCR.

### Primer and probe design

A literature search was conducted to identify species- and genus-specific real-time PCR assays. These assays were re-evaluated for coverage and specificity in silico using the amplicons as queries in BLAST searches in the nucleotide collection (nr/nt) and whole genome shotgun (wgs) databases at NCBI. PCRs were discarded when homologies at the probe sequence or at the 3' end of the primer sequences were > 90% in a non-target organism. Acceptable PCRs were then tested for coverage of all available sequences of the target organism. New PCR targets were selected using MultiMPrimer3 (http://bioinfo.ut.ee/multimprimer3/) or from alignments of gene sequences retrieved from GenBank (Koressaar et al., 2009. Bioinformatics 25: 1349-55). Sequences with adequate in silico coverage and specificity were used for primer and probe design for real-time PCR assays using Primer Express Software 3.0 (Life Technologies, Bleiswijk, The Netherlands).

### Real-time PCR assay

All PCR reactions were performed on a LightCycler 480II (Roche Diagnostics, Almere, The Netherlands). Reaction mixtures contained 12.5 ul of 2x LightCycler 480 Probes Master (Roche Diagnostics, Almere, The Netherlands), 2.5 µl primers and probe(s) and 10 µl DNA input, resulting in a final reaction volume of 25 µl. The optimal primer and probe concentrations were found to range between 300 nM to 900 nM for the primers and 150 and 200 nM for the probes. For the E. coli specific assay the LightCycler Master mix was replaced by the 2x QuantiFast Multiplex PCR Master Mix (Qiagen, Venlo, The Netherlands) since this mix resulted in lower background signals [unpublished data].

Cycling conditions were as follows: 10 min at 95°C followed by 45 cycles of 95°C for 15 sec and 60°C for 1 min. Results were analyzed with the LightCycler Software version 1.5.0. For the multiplex assays, color compensation was applied to each analysis as described in the LightCycler Operator Manual to correct for fluorescent bleed-through. Each experiment included a no-template control (NTC) (PCR grade water) and a positive control (1000 cfu eq of DNA) for control of contamination and amplification. The primer sets and dual labeled hydrolysis probes were
165 obtained from Isogen (De Meern, The Netherlands) and Macrogen (Amsterdam, The Netherlands).

### Analytical performance characteristics of the monoplex assays

The analytical specificity of the monoplex assays was verified using genomic DNA from a panel of bacteria and fungi obtained from pure cultures (Table 3) at a concentration of approximately 1000 cfu eq per reaction for bacteria and 10000 cfu eq for fungi. A total of 15 strains of each target species or genus were used to evaluate the coverage of the assays. The limit of detection (LOD) was determined by testing serial dilutions (10000, 1000, 100, 50, 10, 1 cfu eq/reaction) of purified genomic DNA from two isolates, each performed in duplicate (i.e. 4 reactions per concentration). The LOD was defined as the lowest concentration that was detected in all four reactions. For E. coli a sample was regarded positive when the Cq value (quantification cycle) of the sample was at least two values lower than that of the NTC. The analytical performance of the assays was further explored by calculating the efficiency and linearity (R2179) from these dilution series with the LightCycler software.

To evaluate the performance of the assays for blood-derived bacterial DNA, bacteria were spiked in 200 µl EDTA anticoagulated-blood from healthy adult volunteers such that 1000, 100, 10 and 1 cfu eq per PCR could be evaluated (this equals 50000, 5000, 500 and 50 cfu/ml blood). Results 183 were compared to DNA isolated from bacteria suspended in PBS for 1000 and 100 cfu eq per PCR.

### Analytical performance characteristics of the multiplex assay

The sensitivity of the assays in multiplex and monoplex format was compared using serial dilutions of genomic DNA from pure cultures (1000, 100, 50, 10, 5, 1 cfu eq/reaction) performed in triplicate (fivefold at 1 cfu eq/reaction). Pooled blood samples (200 µl EDTA anticoagulated-blood) from 10 healthy adult volunteers were subjected to the multiplex PCR assay to test for aspecific amplification signals.

### Performance of the multiplex assay on clinical samples

Whole blood samples (200 µl collected aseptically in EDTA-tubes) obtained together with blood culture from 20 subsequent episodes of suspected LOS in preterm infants admitted to the NICU of our hospital were tested with the multiplex assay. Results were compared to blood culture. Approval was obtained from the local medical ethics committee and written informed consent was obtained from the parents or legal guardians of the neonates.

**Table 3**

| List of bacterial strains used to evaluate the specificity of the PCR assays | |
|---|---|
| **Sepsis pathogens** | |
| *Acinetobacter spp.* | Clinical strain^{a} |
| *Acinetobacter baumannii* | ATCC 19606 |
| *Bacteroides fragilis* | ATCC 25282 |
| *Bacillus cereus* | ATCC 11145 |
| *Candida albicans* | ATCC 90028 |
| *Candida glabrata* | ATCC 15545 |
| *Candida parapsilosis* | ATCC 22019 |
| *Citrobacter freundii* | ATCC 8090 |
| *Eikenella corrodens* | DSM 8340 |
| *Enterobacter aerogenes* | ATCC 13048 |
| *Enterobacter cloacae* | ATCC 13047 |
| *Enterobacter asburiae* | Clinical strain^{b} |
| *Enterococcus faecalis* | ATCC 29212 |
| *Enterococcus faecium* | CDC NY2 |
| *Escherichia coli* | ATCC 11775 |
| *Escherichia coli* | ATCC 25922 |
| *Gardnerella vaginalis* | ATCC 14018 |
| *Haemophilus influenzae* | ATCC 49247 |
| *Klebsiella pneumoniae* | ATCC 13883 |
| *Klebsiella oxytoca* | ATCC 13182 |
| *Listeria monocytogenes* | **ATCC 15313** |
| *Morganella morganii* | Clinical strain^{a} |
| *Moraxella catarrhalis* | Clinical strain^{a} |
| *Neisseria meningitidis* | Clinical strain^{a} |
| *Pseudomonas aeruginosa* | **ATCC 27853** |
| *Pseudomonas aeruginosa* | ATCC 10145 |
| *Serratia marcescens* | ATCC 13880 |
| *Serratia marcescens* | DSM 12481 |
| *Staphylococcus aureus* | ATCC 12600 |
| *Staphylococcus aureus* | ATCC 25923 |
| *Staphylococcus epidermidis* | ATCC 14990 |
| *Stenotrophomonas maltophilia* | ATCC 13637 |
| *Streptococcus agalactiae* | DSM 2134 |
| *Streptococcus pneumoniae* | ATCC 49619 |
| *Streptococcus pyogenes* | ATCC 19615 |
| *Streptococcus oralis* | DSM 206267 |
| *Streptococcus parasanguinis* | DSM 6778 |

| **Contaminants of blood culture and skin microbiota** | |
|---|---|
| *Clostridium bifermentans* | DSM 14994 |
| *Corynebacterium xerosis* | ATCC 373 |
| *Lactobacillus acidophilus* | DSM 20079 |
| *Propionibacterium acnes* | DSM 16379 |
| *Veillonella parvula* | DSM 2008 |

| **Additional strains used to evaluate the Streptococcus agalactiae PCR** | |
|---|---|
| *Abiotrofia defectiva* | Clinical strain^{b} |
| *Lactococcus lactis* | **DSM 20481** |
| *Streptococcus dysgalactiae* | Clinical strain^{c} |
| *Streptococcus bovis* | Clinical strain^{c} |
| *Streptococcus salivarius* | Clinical strain^{c} |

| **Additional strains used to evaluate the *E*. *coli, Klebsiella* spp., *P. aeruginosa, S. marcescens* PCR** | |
|---|---|
| *Cronobacter spp.* | Clinical strain^{a} |
| *Proteus mirabilis* | DSM 4479 |
| *Pseudomonas fluorescens* | ATCC 13525 |
| *Pseudomonas putida* | ATCC 12633 |
| *Salmonella enterica* | ATCC13076 |
| *Shigella flexneri* | ATCC 12022 |
| *Yersinia enterocolitica* | ATCC 23715 |

| **Additional strains used to evaluate the *Serratia marcescens* PCR** | |
|---|---|
| *Serratia odorifera* | DSM 4582 |
| *Streptococcus salivarius* | Clinical strain^{c} |

| **Additional strains used to evaluate the *E*. *coli, Klebsiella* spp., *P. aeruginosa, S. marcescens* PCR** | |
|---|---|
| *Serratia odorifera* | DSM 4582 |

| | |
|---|---|
| a Species identification with standard laboratory methods and confirmed with MALDI-TOF VitekMS. ^{b} Kindly provided by dr. A.C. Fluit from the Department of Medical Microbiology, University Medical Center Utrecht. Identification of species was performed as described.(Paauw et al. 2008. PLoS One.3:e3018). ^{c} Kindly provided by Prof. Peter W.M. Hermans, Radboud University Nijmegen Medical Centre. Identification of species was performed by 16S rDNA sequencing. | |

### Results

### Design of the pathogen specific assays

The most prevalent bacterial pathogens causing LOS in VLBW neonates admitted to the NICU were selected for inclusion in the multiplex assay (Table 4), designated the NeoSep-ID. Based on epidemiological data this selection covers almost 90% of sepsis cases caused by bacterial infections (Stoll et al. 1996. J Pediat 129: 63-7; Stoll et al. 2002. Pediatrics 110: 285-291; van den Hoogen et al. 2010. Neonatology. 97: 22-8; Hornik et al. 2012. Early Hum Dev. 88 Suppl 2: S69-74).

Available real-time PCR assays reported in the literature were used for species-specific detection of *Enterococcus faecalis, Staphylococcus aureus* and *Escherichia coli* because re-evaluation in silico of these assays yielded high coverage and specificity (Huijsdens et al. 2002. J Clin Microbiol. 40: 4423-7; Santo Domingo et al. 2003. Biotechnol Lett 25: 261-5; Peters et al. 2007. J Clin Microbiol 45: 3641-6). Searches with the Sa442 sequence (Martineau et al. 1998. J Clin Microbiol 36: 618-23) which has been extensively used for detection of *S. aureus* yielded only a few strains that would remain undetected due to sequence divergence (Klaassen et al. 2003. J Clin Microbiol 41: 4493). Since more than 350 strains showed perfect matches for primers and probe we decided to use the SA442 target for detection. Re-evaluation of the assay used for detection of *E. coli* revealed a 100% match with *Shigella* spp..*Shigella* spp. is rarely encountered in neonatal sepsis and therefore not likely to cause misidentification.

**Table 4. Design of the NeoSep-ID multiplex PCR assay**

| **Dye** | **Reaction 1** | **Reaction 2** | **Reaction 3** |
|---|---|---|---|
| ATTO 425 | *Staphylococcus aureus* | *Escherichia coli* | *Staphylococcus* spp. |
| Fam | *Enterococcus faecalis* | | |
| Yakima Yellow | *Klebsiella* spp. | *Pseudomonas aeruginosa* | |
| ROX | *Streptococcus agalactiae* | *Serratia marcescens* | |
| ATTO 647N | Internal control (PhHV) | | |

A genus specific PCR to identify staphylococci at the genus level based on a previously published assay was adjusted to detect the most important species (*S. epidermidis, S. hominis, S. haemolyticus, S. saprophyticus, S. capitis*) (Rood et al. 2011. Transfusion 51: 2006-11). Samples positive with the genus PCR and negative for *S. aureus* specific testing were considered positive for coagulase negative staphylococci (CoNS). For *Streptococcus agalactiae* the highly specific *cfb* gene was selected and primers and probes were modified to suit our PCR protocol (Ke et al. 2000. Clin Chem 46: 324-31).

MultiMprimer3 was employed to identify novel targets for *Pseudomonas aeruginosa* and *Klebsiella* spp. (using genome sequences of *K. pneumoniae* and *K. oxytoca*) as no existing PCR assay with *adequate in silico* performance was available from literature. MultiMPrimer3 searches for multi-copy species-specific PCR targets by genome comparisons among assembled sequenced genomes. Since insufficient numbers of sequenced and assembled genomes were available for *Serratia marcescens* to use the MultiMPrimer3 tool, we designed specific primers and probes based on multiple alignments of gyrB sequences deposited in GenBank.

Selected targets with their primer and probe sequences are presented in Table 5.

**Table 5**

| Oligonucleotides used in this study. | | | | | |
|---|---|---|---|---|---|
| Species | Primer/Probe | Sequence | Target sequence | Amplicon size (bp) | Reference |
| *Enterococcus faecalis* | Forward primer | 5'-CGCTTCTTTCCTCCCGAGT-3' | 16S rRNA | 143 | (Santo Domingo et al. (2003). Biotechnol Lett. 25: 261-5) |
| | Reverse primer | 5'-GCCATGCGGCATAAACTG-3' | | | |
| | Probe | 5'-CAATTGGAAAGAGGAGTGGCGGACG-3' | | | |
| *Escherichia coli* | Forward primer | 5'-CATGCCGCGTGTATGAAGAA-3' | 16S | 96 | (Huijsdens et al. (2002). J Clin Microbiol. 40: 4423-7) |
| | Reverse primer | 5'-CGGGTAACGTCAATGAGCAAA-3' | | | |
| | Probe | 5'-TATTAACTTTACTCCCTTCCTCCCCGCTGAA-3' | | | |
| *Klebsiella* spp. | Forward primer | 5'-AACCAGGCGTCGATAAT-3' | rhaArhaD operon | 107/108 | |
| | Reverse primer | 5'-GTTTACGGCGCAATCC-3' | | | |
| | Probe | 5'-ACAGGAAAGACAAGACTATGCAGACC-3' | | | |
| *Pseudomonas aeruginosa* | Forward primer | 5'-GCCGAGGTCATGGAATTC-3' | phzE gene | 89 | |
| | Reverse primer | 5'-ATCCGCGCCATCATCTTC-3' | | | |
| | Probe | CGACAACCGCAAGGAAGCCGA-3' | | | |
| *Serratia marcescens* | Forward primer | 5'-GACCGTGAAGACCACTTCCATTAC-3' | gyrB gene | 125 | |
| | Reverse primer | 5'-ACGCCGATGTCGTCTTTCAC-3' | | | |
| | Probe | 5'-CGATCCACCCGAACGTGTTCTACTTCTC-3' | | | |
| *Staphylococcus spp.* | Forward primers | 5'-CCAACTCCAGAACGTGATTCTG-3' | tuf gene | 222 | Adjusted from Rood et al. 2011. |
| | | 5'-CCAACTCCAGAACGTGACTCTG-3' | | | |
| | | 5'-CCAACACCAGAACGTGATTCTG-3' | | | |
| | Reverse primers | 5'-GTTGTCACCAGCTTCAGCGTAGT-3' | | | Transfusion5 1: 2006-11 |
| | | 5'-GTTATCACCAGCTTCAGCGTAAT-3' | | | |
| | | 5'-GTTGTCACCAGCTTCAGCATAGT-3' | | | |
| | Probe | 5'-ACAGGCCGTGTTGAACGTGGKCAAATCAA-3' | | | |
| *Staphylococcus aureus* | Forward primer | 5'-CATCGGAAACATTGTGTTCTGTATG-3' | Sa442 | 94 | Peters et al. (2007). J Clin Microbiol. 45: 3641-6 |
| | Reverse primer | 5'-TTTGGCTGGAAAATATAACTCTCGTA-3' | | | |
| | Probe | 5'- AAGCCGTCTTGATAATCTTTAGTAGTACCGAAG CTGGT-3' | | | |
| *Streptococcus agalactiae* | Forward primer | 5'-TTCACCAGCTGTATTAGAAGTACATGC-3' | cfb gene | 150 | Adjusted from Ke et al. (2000). Clin Chem. 46: 324-31. |
| | Reverse primer | 5'-CCCTGAACATTATCTTTGATATTTCTCA-3' | | | |
| | Probe | 5'-CAAGCCCAGCAAATGGCTCAAAAGCT-3' | | | |
| Internal Control PhHV-1 | Forward primer | 5'-GGGCGAATCACAGATTGAATC-3' | gB gene | 89 | van Doornum et all. (2003). J Clin Microbiol 41: 576-80. |
| | Reverse primer | 5'-GCGGTTCCAAACGTACCAA-3' | | | |
| | Probe | 5'-TTTTTATGTGTCCGCCACCATCTGGATC-3' | | | |

### Analytical performance of the monoplex assays

The specificity of the monoplex assays was assessed using a panel of bacteria and fungi that cause neonatal sepsis or that are regular contaminants of blood culture or skin bacteria (Table 3). In addition, phylogenetically related species and species with target sequence similarity observed with BLAST were tested. Amplification signals were observed only for the target species except in case of the *E. coli* specific PCR. In the *E. coli* assay, amplification signals were observed for *Shigella flexneri* as anticipated from the *in silico* analysis. NTC signals were only present in the *E. coli* specific PCR probably resulting from residual *E. coli* DNA in the Taq polymerase preparation. This signal was reduced significantly (3 Cq values, which is the fractional cycle number (Cq) that is required to reach a quantification threshold) when using the QuantiFast Multiplex PCR Master Mix while the *E. coli* specific signal was not negatively affected [data not shown]. The observed Cq values of the NTC ranged from ±36 to undetectable. Hence all monoplex assays were regarded as specific.

Clinical strains, selected on the basis of differences in AFLP typing patterns and thus representing a set of highly divergent strains, were used to determine the coverage of the assays. For each assay 15 tested strains were successfully detected, resulting in 100% coverage. The LOD, the lowest concentration that was detected in all four reactions, ranged between 1 and 10 genome copies per reaction for all assays and linearity was observed in all assays up to 10 cfu eq/reaction (Table 6). The performance of the assays was further verified with bacteria spiked in whole blood at different concentrations.

**Table 6 Performance characteristics of the monoplex assays.**

| Assay | Analytical sensitivity | | Analytical specificity | Accuracy | |
|---|---|---|---|---|---|
| | LOD in cfu eq/reaction (median Cq value) | Cover age ^{a} (%) | Cross reactivity | Linearity^{b} (R²) | Efficiency^{b} |
| *E. faecalis* | 10 (37.6) | 100 | - | 0.98 | 2.02 |
| *E. coli* | 10 (33.5) | 100 | *Shigella flexneri* | 0.99 | 1.97 |
| *Klebsiella spp.* | 10 (36.9) | 100 | - | 0.98 | 1.99 |
| *P. aeruginosa* | 10 (36.7) | 100 | - | 0.99 | 1.81 |
| S. *marcescens* | 10 (36.2) | 100 | - | 0.92 | 2.07 |
| *Staphylococcus spp.* | 10 (40.0) | 100 | - | 0.98 | 1.98 |
| *S. aureus* | 10 (35.9) | 100 | - | 0.98 | 2.07 |
| *S. agalactiae* | 1 (38.1) | | - | 0.94 | 1.78 |

| | | | | | |
|---|---|---|---|---|---|
| a Calculated from 15 strains ^{b} Median calculated from a dilution series of two strains | | | | | |

The observed detection limits were similar to those obtained with DNA obtained from pure cultures. The PCR curves of bacterial DNA isolated from PBS versus blood coincided virtually completely, demonstrating that no significant PCR-inhibiting factors are co-purified in the current procedure (Fig. 1).

### Design & validation of the multiplex assay

Since most real-time PCR platforms are able to detect fluorescence at 5 different emission wavelength windows (channels), we combined monoplex PCRs to a maximum of five. The sensitivity of the *Staphylococcus* genus assay was greatly reduced in any multiplexed format. This assay therefore was placed in a dedicated reaction, resulting in a total of three reactions (Table 4). The internal control (IC) probe, for detection of PhHV-1 DNA, was designed to emit in the channel with the highest wavelength, as fluorescence is usually lower at highest wavelengths. This ensures that the IC assay will suffer most from potential inhibition in the reaction. In the same vein, the probes detecting the most prevalent bacteria (*E. coli, S. aureus,* CoNS) causing LOS were designed to emit in the lowest wavelength channels. This also positions them in separate reactions (Table 4) reducing the chances that multiple targets will be amplified in a single reaction. In the multiplex assays primers were used at 900nM and probes at 200nM since this resulted in highest fluorescence. Color compensation was applied to correct for fluorescent bleed-through.

Comparison of monoplex (Figure 1) and multiplex assays showed lower fluorescence levels and slightly more variable Cq values for the multiplex assays, but sensitivities were not negatively affected (Table 7). The LODs ranged between 1 and 10 cfu eq/reaction for all multiplex assays (except for *E. coli,* which was 1 cfu eq/reaction). The presence of specific amplification signals coming from blood (e.g. proteins, human leucocyte DNA) was evaluated using 10 pooled blood samples from healthy volunteers. None of the samples yielded positive PCR signals in the multiplex assay except for the IC signal.

### Evaluation of the Multiplex Assay in Clinical Samples

As a proof of principle, EDTA blood samples from 20 subsequent episodes of suspected LOS in preterm neonates were tested in the multiplex assay and compared to blood culture. Nine episodes were positive with both tests (8 CoNS, 1 *E.coli*), 7 were negative; blood culture was positive in case of 3 CoNS infections where PCR was negative. In one episode blood culture detected CoNS and *K. pneumoniae* whereas PCR detected CoNS only. The Cq values ranged between 28.6 and 37.1. As such, concordance was 80% between blood culture and PCR, but there was no significant difference in yield between techniques (McNemar test p-value> 0.05).

**Table 7 Comparison between multiplex and monoplex PCR assays.**

| **Target** | **Assay format** | **50 cfu eq** | **10 cfu eq** | **5 cfu eq** | **1 cfu eq** |
|---|---|---|---|---|---|
| *E. faecalis* | Multiplex | +++ | +++ | +++ | +++++ |
| | Monoplex | +++ | +++ | +++ | +++++ |
| *E. coli* | Multiplex | +++ | ++* | *** | ***** |
| | Monoplex | +++ | +++ | *** | ***** |
| *Klebsiella spp.* | Multiplex | +++ | +++ | +++ | +++++ |
| | Monoplex | +++ | +++ | +++ | ++++- |
| *P. aeruginosa* | Multiplex | +++ | +++ | +++ | ++--- |
| | Monoplex | +++ | +++ | +++ | ++++- |
| *S. marcescens* | Multiplex | +++ | +++ | +++ | ++++- |
| | Monoplex | +++ | +++ | +++ | +++++ |
| *Staphylococcus aureus* | Multiplex | +++ | +++ | +++ | +---- |
| | Monoplex | +++ | +++ | +++ | - |
| *S. agalactiae* | Multiplex | +++ | +++ | +++ | +---- |
| | Monoplex | +++ | ++- | ++- | ++--- |

Different amounts of DNA, indicated on the top row in cfu eq/reaction, were tested in monoplex and multiplex assays. The outcome of individual assays is indicated by + (positive PCR signal); - (no PCR signal) or * (difference with negative control <2 Cq values).

### Example 2

### Materials and Methods

Species and resistance markers to be included were selected based on prevalence in blood cultures of a large group of patients of the AMC Amsterdam, UMC Utrecht and Radboud UMC Nijmegen. A literature search was conducted to identify species- and genus-specific real-time PCR assays. These assays were re-evaluated for coverage and specificity in silico using the amplicons as queries in BLAST searches in the nucleotide collection (nr/nt) and whole 1 genome shotgun (wgs) databases at NCBI. PCRs were discarded when homologies at the probe sequence or at the 3' end of the primer sequences were > 90% in a non-target organism. Acceptable PCRs were then tested for coverage of all available sequences of the target organism. New PCR targets were selected using MultiMPrimer3 (http://bioinfo.ut.ee/multimprimer3/) or from alignments of gene sequences retrieved from GenBank (Koressaar et al. 2009. Bioinformatics 25: 1349-55). Sequences with adequate in silico coverage and specificity were used for primer and probe design for real-time PCR assays using Primer Express 149 Software 3.0 (Life Technologies, Bleiswijk, The Netherlands).

All PCR reactions were performed on a LightCycler 480II (Roche Diagnostics, Almere, The Netherlands). Reaction mixtures contained 12.5 µl of SensimixII (Bioline), 2.5 µl primers and probe(s) and 10 µl DNA input, resulting in a final reaction volume of 25 µl. The optimal primer and probe concentrations were determined and found to range between 300 nM to 900 nM for the primers and 150 and 200 nM for the probes. Cycling conditions were as follows: 10 min at 95°C followed by 45 cycles of 95°C for 15 sec and 60°C for 1 min. Results were analyzed with the LightCycler Software version 1.5.0. For the multiplex assays, color compensation was applied to each analysis as described in the LightCycler Operator Manual to correct for fluorescent bleed-through. Each experiment included a no-template control (NTC) (PCR grade water) and a positive control (1000 cfu eq of DNA) for control of contamination and amplification.

The sensitivity of the multiplex PCR was evaluated by spiking pathogens in different concentrations in blood. Pathogen DNA was isolated from 5 ml of blood using the Polaris procedure (Loonen et al., 2013. PLoS One 8, e72349) followed by easyMag automated DNA extraction. This DNA was used in multiplex and monoplex PCR formats to assess sensitivity.

Internal amplification controls (IAC) were constructed for each multiplex, such that a forward primer from one reaction and a reverse primer from another reaction will produce an amplicon that contains an artificial sequence serving as a hydrolysis probe sequence.

### Results

As an example, results from tests Gram-positive and Gram-negative bacteria are shown in Figure 2. As is indicated in this figure, a-specific signals obtained upon addition of Gram-negative bacterial DNA to the Gram-positive real time PCR reaction (Fig. 2A), or of Gram-positive bacterial DNA to the Gram-negative real time PCR reaction (Fig. 2B), are always lower than the negative control samples, meaning they will not be unjustly interpreted as positive.

The sensitivity of the multiplex PCR is shown in Figure 3 and in Table 8. The limit of detection, indicated as the lowest concentration that was detected in a reaction, of most bacteria was found to be about 1 cfu-equivalent per PCR reaction. The limit of detection of *Candida* spp. was found to be about 10 cfu-equivalent per PCR reaction.

**Table 8. Sensitivity of multiplex BSI PCR for blood-derived pathogen DNA**

| PCR target | Detection limit in monoplex | Detection limit in multiplex |
|---|---|---|
| *Escherichia coli* | 1 cfu / PCR | 1 cfu / PCR |
| *Acinetobacter baumannii* | 1 cfu / PCR | 1 cfu / PCR |
| *Enterococcus faecium* | 1 cfu / PCR | 1 cfu / PCR |
| *Staphylococcus aureus* | 1 cfu / PCR | 1 cfu / PCR |
| *Enterococcus faecalis* | 1 cfu / PCR | 1 cfu / PCR |
| *Pseudomonas aeruginosa* | 1 cfu / PCR | 10 cfu / PCR |
| *Streptococcus pneumoniae* | 1 cfu / PCR | 1 cfu / PCR |
| *Staphylococcus species* | 1 cfu / PCR | 10 cfu / PCR |
| *Klebsiella species* | 1 cfu / PCR | 1 cfu / PCR |
| *Enterococcus species* | 10 cfu /PCR | 10 cfu /PCR |
| *Candida albicans* | 10 cfu /PCR | 10 cfu /PCR |
| *Candida glabrata* | 10 cfu /PCR | 10 cfu /PCR |
| *Candida krusei* | 10 cfu /PCR | 10 cfu /PCR |
| *Aspergillus* | 100 conidia/ PCR | 100 conidia/ PCR |

The sensitivity of the pan-Aspergillus PCR was tested by adding DNA obtained from pure *Aspergillus* cultures to the PCR reactions. Results are shown in Figure 4. Besides positive detection of *Aspergillus candida* (Fig. 4A) and *Aspergillus terreus* (Fig. 4B) also *Aspergillus nidulans, Aspergillus fumigatus, Aspergillus flavus, Aspergillus clavatus,* and *Aspergillus niger* were detected using the pan-Aspergillus PCR assay.

### Blood culture versus multiplex BSI PCR

Clinical blood samples from critically ill patients admitted to the ICU were used to evaluate the multiplex BSI PCR. PCR results were compared to blood culture results. All PCRs performed in the multiplex format show sensitivities of detection between 1 -10 cfu/PCR.

All positive blood cultures were confirmed by PCR (see Table 9). Additionally, 8 *E. coli* positive PCRs were found that were negative in blood culture. Three of these patients had other cultures (urine, sputum) indicating that these PCR results are not false-positives, and suggesting a higher sensitivity of the PCR, compared to the blood culture.

Given the fact that the time-to-result time for the PCR is only 3 hours, whereas the time-to-result time of the blood culture is at least 24 hours, the PCR test is superior since it would result in much more timely adequate treatment of the patient.

**Table 9.**

| Blood culture result | Concordant PCR result | Discrepant PCR result |
|---|---|---|
| *S. aureus 3x* | *S. aureus 3x* | - |
| *P. aeruginosa* | *P. aeruginosa* | - |
| *E. cloacae 2x* | Gram-neg *2x* | - |
| Negative 59x | Negative 51x | *E. coli* 8x |

### Example 3

### Materials and Methods

Approximately 1300 clinical blood samples were taken simultaneously with blood culture samples from critically ill patients admitted to the ICU's of two different academic medical centers (AMC Amsterdam and UMC Utrecht). The samples were tested in the multiplex BSI PCR using the reactions and conditions indicated herein above. All PCR reactions were performed on a LightCycler 480II (Roche Diagnostics, Almere, The Netherlands). PCR results were compared to standard blood culture results.

### Results

About 800 samples were tested negative both by blood culture and by PCR. Other samples were positive in one or both tests. Table 10 shows an overview of the PCR results of samples with positive blood culture. Most pathogens are correctly detected by PCR. Low concordance may be caused by low pathogen loads which could be improved by increasing blood sample volume. Low concordance may also be caused by contamination of the blood culture, which is often the case for coagulase-negative staphylococci (CoNS).

**Table 10: PCR results of samples with concomitant blood cultures positive for pathogens included in the multiplex BSI PCR panel.**

| BC result | # of BC | # of PCR positive | # of PCR negative | concordance |
|---|---|---|---|---|
| *E. coli* | 5 | 5 | 0 | 100% |
| *S. aureus* | 11 | 11 | 0 | 100% |
| *E. faecium* | 21 | 13 | 8 | 62% |
| *E. faecalis* | 8 | 6 | 2 | 75% |
| *P. aeruginosa* | 9 | 9 | 0 | 100% |
| *S. pneumoniae* | 2 | 2 | 0 | 100% |
| *Acinetobacter* | 1 | 0 | 1 | 0% |
| *Klebsiella* | 3 | 2 | 1 | 67% |
| CoNS | 62 | 25 | 37 | 40% |
| *C. albicans* | 9 | 0 | 9 | 0% |
| *C. glabrata* | 7 | 1 | 6 | 14% |
| *C. krusei* | 0 | | | |
| *Candida spp.* | 1 | 1 | 0 | 100% |
| *Aspergillus* | 0 | | | |
| Gram-positive | 3 | 3 | 0 | 100% |
| Gram-negative | 10 | 7 | 3 | 70% |

| | | | | |
|---|---|---|---|---|
| BC: blood culture. CoNS: coagulase-negative staphylococci | | | | |

Table 11 shows samples with negative blood culture and positive PCR results. Clinical records of the patients were screened for culture results of other clinical samples, i.e. urine, pus, sputum, or blood cultures taken at other time-points. Whenever the PCR result was in accordance with any other culture result, it was scored in the culture+ column. Most patients at the ICU will receive antibiotics which could prevent growth of pathogens in blood culture. In contrast, PCR will also detect dead pathogens. Positive PCRs likely reflect true infection, when the detected pathogen is also found in another clinical sample. Thus, PCR may be more sensitive than blood culture to detect infection.

**Table 11: Positive PCR results of samples with concomitant negative blood cultures.**

| PCR result | Culture + | Culture - |
|---|---|---|
| *E. coli* | 29 | 33 |
| *S. aureus* | 28 | 24 |
| *E. faecium* | 19 | 13 |
| *E. faecalis* | 9 | 9 |
| *P. aeruginosa* | 3 | 36 |
| *S. pneumoniae* | 12 | 3 |
| *Acinetobacter* | 2 | 0 |
| *Klebsiella* | 1 | 2 |
| CoNS | 5 | 12 |
| *C. albicans* | 0 | 0 |
| *C. glabrata* | 0 | 0 |
| *C. krusei* | 0 | 0 |
| *Candida spp.* | 0 | 0 |
| *Aspergillus* | 5 | 5 |
| Gram-positive | 41 | 13 |
| Gram-negative | 13 | 6 |

## Claims

1. A method for detecting microbial blood infections comprising the steps of:
a) performing on a blood sample from a subject suspected of suffering from microbial blood infections, or on a sample of nucleic acids isolated therefrom, a nucleic acid amplification reaction,
b) determining the presence or the amount of amplified nucleic acid produced by said nucleic acid amplification reaction,
wherein said nucleic acid amplification reaction comprises the amplification of the *phzE* gene or *phzE* gene product, or a part thereof, of *Pseudomonas aeruginosa.*

2. The method of claim 1, further comprising the amplification of the *rhaA* gene or *rhaA* gene product, or a part thereof, of *Klebsiella* spp., and/or the *tuf* gene or *tuf* gene product, or a part thereof, of *Staphylococcus* spp.

3. The method according to claim 1 or claim 2, wherein the nucleic acid amplification reaction of the *phzE* gene or *phzE* gene product, or a part thereof, is a PCR reaction using as a forward primer the sequence 5'-GCCGAGGTCATGGAATTC-3', using as a reverse primer the sequence 5'-ATCCGCGCCATCATCTTC-3', and using as a probe the sequence 5'-CGACAACCGCAAGGAAGCCGA-3'; the nucleic acid amplification reaction of the *rhaA* gene or *rhaA* gene product, or a part thereof, is a PCR reaction using as a forward primer the sequence 5'-AACCAGGCGTCGATAAT-3', using as a reverse primer the sequence 5'-GTTTACGGCGCAATCC-3', and using as a probe the sequence 5'-ACAGGAAAGACAAGACTATGCAGACC-3; and the nucleic acid amplification reaction of the *tuf* gene or *tuf* gene product, or a part thereof, is a PCR reaction using as a forward primer the sequences 5'-CCAACTCCAGAACGTGATTCTG-3', 5'-CCAACTCCAGAACGTGACTCTG-3' and 5'-CCAACACCAGAACGTGATTCTG-3', using as reverse primers the sequences 5'-GTTGTCACCAGCTTCAGCGTAGT-3', 5'-GTTATCACCAGCTTCAGCGTAAT-3', and 5'-GTTGTCACCAGCTTCAGCATAGT-3', and using as a probe the sequence 5'-ACAGGCCGTGTTGAACGTGGKCAAATCAA-3'.

4. The method of any one of claims 1-3, wherein said method further comprises the amplification of a gene or gene product, or a part thereof, of at least one microorganism selected from the group consisting of *Enterococcus faecalis, Escherichia coli,* and *Staphylococcus aureus,* as part of the same or a separate nucleic acid amplification reaction,

5. The method of any one of claims 1-4, wherein said subject is a neonate, and wherein said method further comprises the amplification of a gene or gene product, or a part thereof, of at least one microorganism selected from the group consisting of *Streptococcus agalactiae* and *Serratia marcescens,* as part of the same or a separate nucleic acid amplification reaction.

6. The method of claim 5, wherein said method comprises performing 3 separate multiplex PCR assays,
wherein a first amplification reaction comprises the amplification of a gene or gene product, or a part thereof, of *Staphylococcus aureus, Enterococcus faecalas, Klebsaella* spp., and *Streptococcus agalactiae;*
wherein a second amplification reaction comprises the amplification of a gene or gene product, or a part thereof, of *Escherichia coli, Pseudomonas aeruginosa,* and *Serratia marcescens;* and
wherein a third amplification reaction comprises the amplification of a gene or gene product, or a part thereof, of *Staphylococcus* spp.

7. The method of any one of claims 1-4, wherein said subject is a non-neonate, preferably an adult, and wherein said method further comprises the amplification of a gene or gene product, or a part thereof, of at least one of the following targets: a microorganism selected from the group consisting of *Acinetobacter baumannii, Enterococcus faecium, Streptococcus pneumoniae, Enterococcus* spp., *Candida albicans, Candida glabrata, Candida krusei, Aspergillus* spp, Gram positive bacteria, Gram negative bacteria, *Candida* spp. or a gene or gene product selected from *mecA, vanA,* and *ctxM,* as part of the same or a separate nucleic acid amplification reaction.

8. The method of claim 7, wherein said method comprises performing 5 separate multiplex PCR assays,
wherein a first amplification reaction comprises the amplification of a gene or gene product, or a part thereof, of *Aspergillus* spp, Gram positive bacteria, Gram negative bacteria, *Candida* spp. and *Candida glabrata;*
wherein a second amplification reaction comprises the amplification of a gene or gene product, or a part thereof, of *Escherichia coli, Enterococcus faecium, Acinetobacter baumannii,* and the *mecA* gene;
wherein a third amplification reaction comprises the amplification of a gene or gene product, or a part thereof, of *Staphylococcus aureus, Enterococcus faecalis, Pseudomonas aeruginosa, Candida krusei,* and *Streptococcus pneumoniae;*
wherein a fourth amplification reaction comprises the amplification of a gene or gene product, or a part thereof, of *Staphylococcus* spp., *Enterococcus* spp., *Klebsiella* spp. and *Candida albicans,* and
wherein a fifth amplification reaction comprises the amplification of a gene or gene product, or a part thereof, of *vanA,* and *ctxM.*

9. The method of any one of claims 7 or 8, wherein said gene of *Escherichia coli* is the *gadA* and/or *gadB* gene and wherein said nucleic acid amplification reaction is a real-time PCR reaction using as a forward primer the sequence 5'-GGCTTCGAAATGGACTTTGCT-3', using as reverse primer the sequence 5'-TGGGCAATACCCTGCAGTTT-3', and using as a probe the sequence 5'-CTGTTGCTGGAAGACTACAAAGCCTCCCTG-3'.

10. The method of any one of claims 7-9, wherein said gene of *Enterococcus faecalis* is the *ref12A* gene, and wherein said nucleic acid amplification reaction is a real-time PCR reaction using as a forward primer the sequence 5'-ATGCGTCTCGTCACAGTA-3', using as reverse primer the sequence 5'-GGTACGATGATTTCATCTGT-3', and using as a probe the sequence 5'-AGTTGCGATGTTTCACTGTGAAGCA-3'.

11. The method of any one of claims 7-10, wherein said gene of *Streptococcus pneumoniae* is the *comX* gene and wherein said nucleic acid amplification reaction is a real-time PCR reaction using as a forward primer the sequence 5'-GGTCTCTGGCTAGATGATTATTATCTCTT-3', using as reverse primer the sequence 5'-ATAGTAAACTCCTTAAACACAATGCGTAA-3', and using as a probe the sequence 5'-CGCCCTCGAAATCGTTCATTGCTTAAGA-3'.

12. The method of any one of claims 7-11,
- wherein said gene of *Aspergillus* spp is the *18S-28S rRNA* ITS region and wherein said nucleic acid amplification reaction is a real-time PCR reaction using as a forward primer the sequence 5'-GCGTCATTGCTGCCCTCAAGC-3', using as reverse primer the sequence 5'-ATATGCTTAAGTTCAGCGGGT-3', and using as a probe the sequence 5'-CCTCGAGCGTATGGGGC-3'; and/or
- wherein said gene of Gram positive bacteria is the 16S rDNA gene and wherein said nucleic acid amplification reaction is a real-time PCR reaction using as a forward primer the sequence 5'-TGGAGCATGTGGTTTAATTCGA-3', using as reverse primer the sequence 5'-TGCGGGACTTAACCCAACA-3', and using as a probe the sequence 5'-TGGTGCATGGTTG-3'; and/or
- wherein said gene of Gram negative bacteria is the 16S rDNA gene and wherein said nucleic acid amplification reaction is a real-time PCR reaction using as a forward primer the sequence 5'-TGGAGCATGTGGTTTAATTCGA-3', using as reverse primer the sequence 5'-TGCGGGACTTAACCCAACA-3', and using as a probe the sequence 5'-TGCTGCATGGCTGT-3'; and/or
- wherein said gene of *Candida* spp. is the *18S-28S rRNA* ITS region and wherein said nucleic acid amplification reaction is a real-time PCR reaction using as a forward primer the sequence 5'-CATGCCTGTTTGAGCGTCRTTT-3', using as reverse primer the sequence 5'-ATATGCTTAAGTTCAGCGGGT-3', and using as a probe the sequence 5'-TCGTATTGCTCAACACCAAACCC-3'; and/or
- wherein said gene of *Candida glabrata* is the *18S-28S rRNA* ITS region and wherein said nucleic acid amplification reaction is a real-time PCR reaction using as a forward primer the sequence 5'-CATGCCTGTTTGAGCGTCRTTT-3', using as reverse primer the sequence 5'-ATATGCTTAAGTTCAGCGGGT-3', and using as a probe the sequence 5'-ATCAGTATGTGGGACACGAGCG-3'; and/or
- wherein said gene is the *mecA* gene or a part thereof, and wherein said nucleic acid amplification reaction is a real-time PCR reaction using as a forward primer the sequence 5'-GATCGCAACGTTCAATTTAATTTT-3', using as reverse primer the sequence 5'-GCTTTGGTCTTTCTGCATTCCT-3', and using as a probe the sequence 5'-AATGACGCTATGATCCCAATCTAACTTCCACAT-3'; and/or
- wherein said gene of *Candida krusei* is the *18S-28S rRNA* ITS region and wherein said nucleic acid amplification reaction is a real-time PCR reaction using as a forward primer the sequence 5'-CATGCCTGTTTGAGCGTCRTTT-3', using as reverse primer the sequence 5'-ATATGCTTAAGTTCAGCGGGT-3', and using as a probe the sequence 5'-ACGACGTGTAAAGAGCGTCGG-3'; and/or
- wherein said gene of *Enterococcus* spp. is the 23S rDNA gene and wherein said nucleic acid amplification reaction is a real-time PCR reaction using as a forward primer the sequence 5'-TGCGGGGATGAGGTGTG-3', using as reverse primer the sequence 5'-CAAACAGTGCTCTACCTCCATCAT-3', and using as a probe the sequence 5'-TAGCCCTAAAGCTATTTCGGAGAGAACCA-3'; and/or
- wherein said gene of *Candida albicans* is the *18S-28S rRNA* ITS region and wherein said nucleic acid amplification reaction is a real-time PCR reaction using as a forward primer the sequence 5'-CATGCCTGTTTGAGCGTCRTTT-3', using as reverse primer the sequence 5'-ATATGCTTAAGTTCAGCGGGT-3', and using as a probe the sequence 5'-TAAGGCGGGATCGCTTTGACA-3'; and/or
- wherein said gene is the *vanA* gene or a part thereof, and wherein said nucleic acid amplification reaction is a real-time PCR reaction using as a forward primer the sequence 5'-CGGTTTCACGTCATACAGTCGTT-3', using as reverse primer the sequence 5'-CAGTTCGGGAAGTGCAATACC-3', and using as a probe the sequence 5'-TCCCCGTATGATGGCCGCTGC-3'; and/or
- wherein said gene is the *ctxM-1* gene or a part thereof, and wherein said nucleic acid amplification reaction is a real-time PCR reaction using as a forward primer the sequence 5'-ATGTGCAGYACCAGTAARGTKATGGC-3', using as reverse primer the sequence 5'-ATCACKCGGRTCGCCNGGRAT-3', and using as a probe the sequence 5'-CCCGACAGCTGGGAGACGAAACGT-3'; and/or
- wherein said gene is the *ctxM-9* gene or a part thereof, and wherein said nucleic acid amplification reaction is a real-time PCR reaction using as a forward primer the sequence 5'-ATGTGCAGYACCAGTAARGTKATGGC-3', using as reverse primer the sequence 5'-ATCACKCGGRTCGCCNGGRAT-3', and using as a probe the sequence 5'-CTGGATCGCACTGAACCTACGCTGA-3'.

13. A kit of parts adapted for performing the method of any one of claims 1-12, said kit comprising at least one forward or reverse primer or probe as defined in any one of claims 2 and 9-12, preferably wherein at least one of said at least one forward or reverse primer or probe comprises a detectable label, preferably a fluorescent label.

14. A method for monitoring sepsis or for determining the efficacy of an anti-sepsis treatment, said method comprising performing a method according to any one of claims 1-13 on at least two samples, wherein said samples are obtained at different time points in the course of the sepsis or at different time points in the course of an anti-sepsis treatment.

15. A specific antibiotic for use in a method of treating a subject suffering or suspected of suffering from sepsis, the method comprising treating a subject with said specific antibiotic wherein in the sample of said subject one or more of *Pseudomonas aeruginosa, Klebsiella species, Escherichia coli, Acinetobacter baumannii, Enterococcus faecalis, Enterococcus faecium, Staphylococcus species, Staphylococcus aureus, Streptococcus pneumoniae, Streptococcus agalactiae, Serratia marcescens, Candida albicans, Candida glabrata, Candida krusei,* pan-*Aspergillus,* pan-*Candida,* Gram-positive bacteria, Gram-negative bacteria, *MecA, VanA,* and/or *CTXM*, was detected with a method according to any one of claims 1-13.

## Patentansprüche

1. Verfahren zur Erkennung mikrobieller Blutinfektionen, umfassend die Schritte von:
a) Durchführen einer Nukleinsäure-Amplifikationsreaktion an einer Blutprobe von einem Patienten, der vermutlich an mikrobiellen Blutinfektionen leidet, oder an einer Probe von daraus isolierten Nukleinsäuren,
b) Bestimmen der Anwesenheit oder der Menge amplifizierter Nukleinsäure, produziert durch die Nukleinsäure-Amplifikationsreaktion,
wobei die Nukleinsäure-Amplifikationsreaktion die Amplifikation des *phzE*-Gens oder *phzE*-Genprodukts oder eines Teils davon von *Pseudomonas aeruginosa* umfasst.

2. Verfahren nach Anspruch 1, ferner umfassend die Amplifikation des *rhaA*-Gens oder *rhaA*-Genprodukts oder eines Teils davon von *Klebsiella* spp. und/oder des *tuf*-Gens oder *tuf*-Genprodukts oder eines Teils davon von *Staphylococcus* spp.

3. Verfahren nach Anspruch 1 oder Anspruch 2, wobei die Nukleinsäure-Amplifikationsreaktion des *phzE*-Gens oder *phzE*-Genprodukts oder eines Teils davon eine PCR-Reaktion ist, unter Verwendung der Sequenz 5'-GCCGAGGTCATGGAATTC-3' als einen Vorwärtsprimer, unter Verwendung der Sequenz 5'-ATCCGCGCCATCATCTTC-3' als einen Rückwärtsprimer und unter Verwendung der Sequenz 5'-CGACAACCGCAAGGAAGCCA-3' als eine Sonde; die Nukleinsäure-Amplifikationsreaktion des *rhaA*-Gens oder *rhaA*-Genprodukts oder eines Teils davon eine PCR-Reaktion ist, unter Verwendung der Sequenz 5'-AACCAGGCGTCGATAAT-3' als einen Vorwärtsprimer, unter Verwendung der Sequenz 5'-GTTTACGGCGCAATCC-3' als einen Rückwärtsprimer und unter Verwendung der Sequenz 5'-ACAGGAAAGACAAGACTATGCAGACC-3' als eine Sonde; und die Nukleinsäure-Amplifikationsreaktion des *tuf*-Gens oder *tuf*-Genprodukts oder eines Teils davon eine PCR-Reaktion ist, unter Verwendung der Sequenzen 5'-CCAACTCCAGAACGTGATTCTG-3', 5'-CCAACTCCAGAACGTGACTCTG-3' und 5'-CCAACACCAGAACGTGATTCTG-3' als einen Vorwärtsprimer, unter Verwendung der Sequenzen 5'-GTTGTCACCAGCTTCAGCGTAGT-3', 5'-GTTATCACCAGCTTCAGCGTAAT-3', und 5'-GTTGTCACCAGCTTCAGCATAGT-3' als einen Rückwärtsprimer und unter Verwendung der Sequenz 5'-ACAGGCCGTGTTGAACGTGGKCAAATCAA-3' als eine Sonde.

4. Verfahren nach einem der Ansprüche 1-3, wobei das Verfahren ferner die Amplifikation eines Gens oder Genprodukts oder eines Teils davon von mindestens einem Mikroorganismus, ausgewählt aus der Gruppe, bestehend aus *Enterococcus faecals, Escherichia coli* und *Staphylococcus aureus,* als Teil derselben oder einer separaten Nukleinsäure-Amplifikationsreaktion.

5. Verfahren nach einem der Ansprüche 1-4, wobei der Patient ein Neugeborener ist und wobei das Verfahren ferner die Amplifikation eines Gens oder Genprodukts oder eines Teils davon von mindestens einem Mikroorganismus, ausgewählt aus der Gruppe, bestehend aus *Streptococcus agalactiae* und *Serratia marcescens* als Teil derselben oder einer separaten Nukleinsäure-Amplifikationsreaktion umfasst.

6. Verfahren nach Anspruch 5, wobei das Verfahren die Durchführung von 3 separaten Multiplex-PCR-Assays umfasst,
wobei eine erste Amplifikationsreaktion die Amplifikation eines Gens oder Genprodukts oder eines Teils davon von *Staphylococcus aureus, Enterococcus faecalis, Klebsiella* spp. und *Streptococcus agalactiae* umfasst;
wobei eine zweite Amplifikationsreaktion die Amplifikation eines Gens oder Genprodukts oder eines Teils davon von *Escherichia coli, Pseudomonas aeruginosa* und *Serratia marcescens* umfasst; und
wobei eine dritte Amplifikationsreaktion die Amplifikation eines Gens oder Genprodukts oder eines Teils davon von *Staphylococcus* spp. umfasst.

7. Verfahren nach einem der Ansprüche 1-4, wobei der Patient ein Neugeborener ist, bevorzugt ein Erwachsener, und wobei das Verfahren ferner die Amplifikation eines Gens oder Genprodukts oder eines Teils davon von mindestens einem der Ziele umfasst: ein Mikroorganismus, ausgewählt aus der Gruppe, bestehend aus *Acinetobacter baumannii*, *Enterococcus faecium, Streptococcus pneumoniae, Enterococcus* spp., *Candida albicans, Candida glabrata, Candida krusei, Aspergillus* spp., grampositiven Bakterien, gramnegativen Bakterien, *Candida* spp. oder ein Gen oder Genprodukt, ausgewählt aus *mecA* und *ctxM,* als Teil derselben oder einer separaten Nukleinsäure-Amplifikationsreaktion.

8. Verfahren nach Anspruch 7, wobei das Verfahren die Durchführung von 5 separaten Multiplex-PCR-Assays umfasst,
wobei eine erste Amplifikationsreaktion die Amplifikation eines Gens oder Genprodukts oder eines Teils davon von *Aspergillus* spp., grampositiven Bakterien, gramnegativen Bakterien, *Candida* spp. und *Candedida glabrata* umfasst;
wobei eine zweite Amplifikationsreaktion die Amplifikation eines Gens oder Genprodukts oder eines Teils davon von *Escherichia coli, Enterococcus faecium, Acinetobacter baumannii* und des *mecA*-Gens umfasst;
wobei eine dritte Amplifikationsreaktion die Amplifikation eines Gens oder Genprodukts oder eines Teils davon von *Staphylococcus aureus, Enterococcus faecalis, Pseudomonas aeruginosa, Candida krusei* und *Streptococcus Pneumoniae* umfasst;
wobei eine vierte Amplifikationsreaktion die Vervielfältigung eines Gens oder Genprodukts oder eines Teils davon von *Staphylococcus* spp., *Enterococcus* spp., *Klebsiella* spp. und *Candida albicans* umfasst, und
wobei eine fünfte Amplifikationsreaktion die Amplifikation eines Gens oder Genprodukts oder eines Teils davon von *vanA,* und *ctxM* umfasst.

9. Verfahren nach einem der Ansprüche 7 oder 8, wobei das Gen von *Escherichia coli* das *gadA-* und/oder *gadB*-Gen ist und wobei die Nukleinsäure-Amplifikationsreaktion eine PCR-Reaktion in Echtzeit ist, unter Verwendung der Sequenz 5'-GGCTTGAAATGGACTTTGCT-3' als ein Vorwärtsprimer, unter Verwendung der Sequenz 5'-TGGGCAATACCCTGCAGTTT-3'-als ein Rückwärtsprimer und unter Verwendung der Sequenz 5'-CTGTTGCTGGAAGACTACAAAGCCTCCCTG-3' als eine Sonde.

10. Verfahren nach einem der Ansprüche 7-9, wobei das Gen von *Enterococcus faecalis* das *ref12A*-Gen ist und wobei die Nukleinsäure-Amplifikationsreaktion eine PCR-Reaktion in Echtzeit ist, unter Verwendung der Sequenz 5'-ATGCCTCTCGTCACAGTA-3' als einen Vorwärtsprimer, unter Verwendung der Sequenz 5'-GGTACCGATGATTTCATCTGT-3' als einen Rückwärtsprimer und unter Verwendung der Sequenz 5'-AGTTGCGATGTTTCACTGTGAAGCA-3' als eine Sonde.

11. Verfahren nach einem der Ansprüche 7-10, wobei das Gen von *Streptococcus pneumoniae* das *comX*-Gen ist und wobei die Nukleinsäure-Amplifikationsreaktion eine PCR-Reaktion in Echtzeit ist, unter Verwendung der Sequenz 5'-GGTCTCTGGCTAGATGATTATTATCTCTT-3' als einen Vorwärtsprimer, unter Verwendung der Sequenz 5'-ATAGTAAACTCCTTAAACACAATGCGTAA-3' als einen Rückwärtsprimer und unter Verwendung der Sequenz 5'-CGCCCTCGAAATCGTTCATTGCTTAAGA-3' als eine Sonde.

12. Verfahren nach einem der Ansprüche 7-11,
- wobei das Gen von *Aspergillus* spp die *18S-28S rRna* ITS-Region ist und wobei die Nukleinsäure-Amplifikationsreaktion eine PCR-Reaktion in Echtzeit ist, unter Verwendung der Sequenz 5'-GCGTCATTGCTGCCCTCAAGC-3' als einen Vorwärtsprimer, unter Verwendung der Sequenz 5'-ATATGCTTAAGTTCAGCGGGT-3' als einen Rückwärtsprimer und unter Verwendung der Sequenz 5'-CCTCGAGCGTATGGGGC-3' als eine Sonde; und/oder
- wobei das Gen von grampositiven Bakterien das 16S rDNA-Gen ist und wobei die Nukleinsäure-Amplifikationsreaktion eine PCR-Reaktion in Echtzeit ist, unter Verwendung der Sequenz 5'-TGGAGCATGTGGTTTAATTCGA-3' als einen Vorwärtsprimer, unter Verwendung der Sequenz 5'-TGCGGGACTTAACCCAACA-3' als einen Rückwärtsprimer und unter Verwendung der Sequenz 5'-TGGTGCATGGTTG-3' als eine Sonde; und/oder
- wobei das Gen von gramnegativen Bakterien das 16S rDNA-Gen ist und wobei die Nukleinsäure-Amplifikationsreaktion eine PCR-Reaktion in Echtzeit ist, unter Verwendung der Sequenz 5'-TGGAGCATGTGGTTTAATTCGA-3' als einen Vorwärtsprimer, unter Verwendung der Sequenz 5'-TGCGGGACTTAACCCAACA-3' als einen Rückwärtsprimer und unter Verwendung der Sequenz 5'-TGCTGCATGGCTGT-3' als eine Sonde; und/oder
- wobei das Gen von *Candida* spp. die *18S-28S rRNA* ITS-Region ist und wobei die Nukleinsäure-Amplifikationsreaktion eine PCR-Reaktion in Echtzeit ist, unter Verwendung der Sequenz 5'-CATGCCTGTTTGAGCGTCRTTT-3' als einen Vorwärtsprimer, unter Verwendung der Sequenz 5'-ATATGCTTAAGTTCAGCGGGT-3' als einen Rückwärtsprimer und unter Verwendung der Sequenz 5'-TCGTATTGCTCAACACCAAACCC-3' als eine Sonde; und/oder
- wobei das Gen von *Candida glabrata* die *18S-28S rRNA* ITS-Region ist und wobei die Nukleinsäure-Amplifikationsreaktion eine PCR-Reaktion in Echtzeit ist, unter Verwendung der Sequenz 5'-CATGCCTGTTTGAGCGTCRTTT-3' als einen Vorwärtsprimer, unter Verwendung der Sequenz 5'-ATATGCTTAAGTTCAGCGGGT-3' als einen Rückwärtsprimer und unter Verwendung der Sequenz 5'-ATCAGTATGTGGGACACGAGCG-3' als eine Sonde; und/oder
- wobei das Gen das *mecA*-Gen oder ein Teil davon ist und wobei die Nukleinsäure-Amplifikationsreaktion eine PCR-Reaktion in Echtzeit ist, unter Verwendung der Sequenz 5'-GATCGCAACGTTCAATTTAATTTT-3' als einen Vorwärtsprimer, unter Verwendung der Sequenz 5'-GCTTTGGTCTTTCTGCATTCCT-3' als einen Rückwärtsprimer und unter Verwendung der Sequenz 5'-AATGACGCTATGATCCCAATCTAACTTCCACAT-3' als eine Sonde; und/oder
- wobei das Gen von *Candida krusei* die *18S-28S rRNA* ITS-Region ist und wobei die Nukleinsäure-Amplifikationsreaktion eine PCR-Reaktion in Echtzeit ist, unter Verwendung der Sequenz 5'-CATGCCTGTTTGAGCGTCRTTT-3' als einen Vorwärtsprimer, unter Verwendung der Sequenz 5'-ATATGCTTAAGTTCAGCGGGT-3' als einen Rückwärtsprimer und unter Verwendung der Sequenz 5'-ACGACGTGTAAAGAGCGTCGG-3' als eine Sonde; und/oder
- wobei das Gen von *Enterococcus* spp. das 28S rDNA-Gen ist und wobei die Nukleinsäure-Amplifikationsreaktion eine PCR-Reaktion in Echtzeit ist, unter Verwendung der Sequenz 5'-TGCGGGGATGAGGTGTG-3' als einen Vorwärtsprimer, unter Verwendung der Sequenz 5'-CAAACAGTGCTCTACCTCCATCAT-3' als einen Rückwärtsprimer und unter Verwendung der Sequenz 5'-TAGCCCTAAAGCTATTTCGGAGAGAACCA-3' als eine Probe; und/oder
- wobei das Gen von *Candida albicans* die *18S-28S rRNA* ITS-Region ist und wobei die Nukleinsäure-Amplifikationsreaktion eine PCR-Reaktion in Echtzeit ist, unter Verwendung der Sequenz 5'-CATGCCTGTTTGAGCGTCRTTT-3' als einen Vorwärtsprimer, unter Verwendung der Sequenz 5'-ATATGCTTAAGTTCAGCGGGT-3' als einen Rückwärtsprimer und unter Verwendung der Sequenz 5'-TAAGGCGGGATCGCTTTGACA-3' als eine Sonde; und/oder
- wobei das Gen das *vanA*-Gen oder ein Teil davon ist und wobei die Nukleinsäure-Amplifikationsreaktion eine PCR-Reaktion in Echtzeit ist, unter Verwendung der Sequenz 5'-CGGTTTCACGTCATACAGTCGTT-3' als einen Vorwärtsprimer, unter Verwendung der Sequenz 5'-CAGTTCGGGAAGTGCAATACC-3' als einen Rückwärtsprimer und unter Verwendung der Sequez 5'-TCCCCGTATGATGGCCGCTGC-3' als eine Sonde; und/oder
- wobei das Gen das *ctxM-1*-Gen oder ein Teil davon ist und wobei die Nukleinsäure-Amplifikationsreaktion eine PCR-Reaktion in Echtzeit ist, unter Verwendung der Sequenz 5'-ATGTGCAGYACCAGTAARGTKATGGC-3' als einen Vorwärtsprimer, unter Verwendung der Sequenz 5'-ATCACKCGGRTCGCCNGGRAT-3' als einen Rückwärtsprimer und unter Verwendung der Sequenz 5'-CCCGACAGCTGGGAGACGAAACGT-3' als eine Sonde; und/oder
- wobei das Gen das *ctxM-9*-Gen oder ein Teil davon ist und wobei die Nukleinsäure-Amplifikationsreaktion eine PCR-Reaktion in Echtzeit ist, unter Verwendung der Sequenz 5'-ATGTGCAGYACCAGTAARGTKATGGC-3' als einen Vorwärtsprimer, unter Verwendung der Sequenz 5'-ATCACKCGGRTCGCCNGGRAT-3' als einen Rückwärtsprimer und unter Verwendung der Sequenz 5'-CTGGATCGCACTGAACCTACGTGA-3' als eine Sonde.

13. Kit von Teilen, angepasst zur Durchführung des Verfahrens nach einem der Ansprüche 1-12, das Kit umfassend mindestens einen Vorwärts- oder Rückwärtsprimer oder eine Sonde, wie definiert in einem der Ansprüche 2 und 9-12, bevorzugt wobei mindestens eines von dem mindestens einen Vorwärts- oder Rückwärtsprimer oder der Sonde ein erkennbares Label, bevorzugt ein fluoreszierendes Label, umfasst.

14. Verfahren zur Überwachung von Sepsis oder zur Bestimmung der Wirksamkeit einer Antisepsisbehandlung, das Verfahren umfassend die Durchführung eines Verfahrens nach einem der Ansprüche 1-13 an mindestens zwei Proben, wobei die Proben zu verschiedenen Zeitpunkten im Verlauf der Sepsis oder zu verschiedenen Zeitpunkten im Verlauf einer Antisepsisbehandlung gewonnen werden.

15. Spezifisches Antibiotikum zur Verwendung in einem Verfahren zur Behandlung eines Patienten, der an Sepsis leidet oder vermutlich leidet, das Verfahren umfassend die Behandlung eines Patienten mit dem spezifischen Antibiotikum, wobei in der Probe des Patienten eines oder mehrere von *Pseudomonas aeruginosa, Klebsiella species, Eschericia coli, Acinetobacter baumannii*, *Enterococcus faecalis, Enterococcus faecium, Staphylococcus species, Staphylococcus aureus, Streptococcus pneumoniae, Streptococcus agalactiae, Serratia marcescens, Candida albicans, Candida glabrata, Candida krusei,* pan-*Aspergillus,* pan-*Candida,* grampositive Bakterien, gramnegative Bakterien, *MecA, VanA* und/oder *CTXM* erkannt wurde mit einem Verfahren nach einem der Ansprüche 1-13.

## Revendications

1. Procédé de détection d'infections microbiennes du sang, comportant les étapes suivantes :
a) réaliser, sur un échantillon de sang provenant d'un sujet suspecté de souffrir d'infections microbiennes du sang ou sur un échantillon d'acides nucléiques isolés à partir de cet échantillon de sang, une réaction d'amplification des acides nucléiques,
b) et déterminer la présence ou la quantité des acides nucléiques amplifiés produits par ladite réaction d'amplification des acides nucléiques,
étant entendu que ladite réaction d'amplification des acides nucléiques comporte l'amplification du gène *phzE* ou du produit du gène *phzE* de *Pseudomonas aeruginosa,* ou d'une partie de ce gène ou de ce produit.

2. Procédé conforme à la revendication 1, qui comporte en outre l'amplification du gène *rhaA* ou du produit du gène *rhaA* de *Klebsiella* spp., ou d'une partie de ce gène ou de ce produit, et/ou l'amplification du gène *tuf* ou du produit du gène *tuf* de *Staphylococcus* ssp., ou d'une partie de ce gène ou de ce produit.

3. Procédé conforme à la revendication 1 ou 2, dans lequel
- la réaction d'amplification d'acides nucléiques du gène *phzE* ou du produit du gène *phzE,* ou d'une partie de ce gène ou de ce produit, est une réaction PCR dans laquelle on utilise comme amorce sens la séquence 5'-GCCGAGGTCATGGAATTC-3', comme amorce anti-sens la séquence 5'-ATCCGCGCCATCATCTTC-3', et comme sonde la séquence 5'-CGACAACCGCAAGGAAGCCGA-3' ;
- la réaction d'amplification d'acides nucléiques du gène *rhaA* ou du produit du gène *rhaA,* ou d'une partie de ce gène ou de ce produit, est une réaction PCR dans laquelle on utilise comme amorce sens la séquence 5'-AACCAGGCGTCGATAAT-3', comme amorce anti-sens la séquence 5'-GTTTACGGCGCAATCC-3', et comme sonde la séquence 5'-ACAGGAAAGACAAGACTATGCAGACC-3' ;
- et la réaction d'amplification d'acides nucléiques du gène *tuf* ou du produit du gène *tuf,* ou d'une partie de ce gène ou de ce produit, est une réaction PCR dans laquelle on utilise comme amorces sens les séquences 5'-CCAACTCCAGAACGTGATTCTG-3', 5'-CCAA-CTCCAGAACGTGACTCTG-3' et 5'-CCAACACCAGAACGTGAT-TCTG-3', comme amorces anti-sens les séquences 5'-GTTGTCACC-AGCTTCAGCGTAGT-3', 5'-GTTATCACCAGCTTCAGCGTAAT-3' et 5'-GTTGTCACCAGCTTCAGCATAGT-3', et comme sonde la séquence 5'-ACAGGCCGTGTTGAACGTGGKCAAATCAA-3'.

4. Procédé conforme à l'une des revendications 1 à 3, lequel procédé comporte en outre l'amplification d'un gène ou d'un produit de gène, ou d'une partie d'un tel gène ou produit, d'au moins un micro-organisme choisi dans l'ensemble constitué par *Enterococcus faecalis, Escherichia coli* et *Staphylococcus aureus,* faisant partie de la même réaction d'amplification ou constituant une réaction séparée d'amplification d'acides nucléiques.

5. Procédé conforme à l'une des revendications 1 à 4, dans lequel ledit sujet est un nouveau-né, et lequel procédé comporte en outre l'amplification d'un gène ou d'un produit de gène, ou d'une partie d'un tel gène ou produit, d'au moins un micro-organisme choisi dans l'ensemble constitué par *Streptococcus agalactiae* et *Serratia marcescens,* faisant partie de la même réaction d'amplification ou constituant une réaction séparée d'amplification d'acides nucléiques.

6. Procédé conforme à la revendication 5, lequel procédé comporte le fait de réaliser séparément trois essais de PCR en mode multiplexe, étant entendu
- qu'une première réaction d'amplification comporte l'amplification d'un gène ou d'un produit de gène, ou d'une partie d'un tel gène ou produit, de *Staphylococcus aureus, Enterococcus faecalis, Klebsiella* spp., et *Streptococcus agalactiae* ;
- qu'une deuxième réaction d'amplification comporte l'amplification d'un gène ou d'un produit de gène, ou d'une partie d'un tel gène ou produit, d'*Escherichia coli, Pseudomonas aeruginosa* et *Serratia marcescens* ;
- et qu'une troisième réaction d'amplification comporte l'amplification d'un gène ou d'un produit de gène, ou d'une partie d'un tel gène ou produit, de *Staphylococcus* spp.

7. Procédé conforme à l'une des revendications 1 à 4, dans lequel ledit sujet n'est pas un nouveau-né, mais est de préférence un adulte, et lequel procédé comporte en outre l'amplification d'un gène ou d'un produit de gène, ou d'une partie d'un tel gène ou produit, d'au moins l'une des cibles suivantes : un micro-organisme choisi dans l'ensemble constitué par *Acinetobacter baumannii, Enterococcus faecium, Streptococcus pneumoniae, Enterococcus* spp., *Candida albicans, Candida glabrata, Candida krusei, Aspergillus* spp., les bactéries Gram-positives, les bactéries Gram-négatives, et *Candida* spp., ou d'un gène ou d'un produit de gène choisi parmi *mecA, vanA* et *ctxM,* faisant partie de la même réaction d'amplification ou constituant une réaction séparée d'amplification d'acides nucléiques.

8. Procédé conforme à la revendication 7, lequel procédé comporte le fait de réaliser séparément cinq essais de PCR en mode multiplexe, étant entendu
- qu'une première réaction d'amplification comporte l'amplification d'un gène ou d'un produit de gène, ou d'une partie d'un tel gène ou produit, d'*Aspergillus* spp., de bactéries Gram-positives, de bactéries Gram-négatives, de *Candida* spp., et de *Candida glabrata* ;
- qu'une deuxième réaction d'amplification comporte l'amplification d'un gène ou d'un produit de gène, ou d'une partie d'un tel gène ou produit, *d'Escherichia coli, Enterococcus faecium* et *Acinetobacter baumannii,* et du gène *mecA* ;
- qu'une troisième réaction d'amplification comporte l'amplification d'un gène ou d'un produit de gène, ou d'une partie d'un tel gène ou produit, de *Staphylococcus aureus, Enterococcus faecalis, Pseudomonas aeruginosa, Candida krusei,* et *Streptococcus pneumoniae* ;
- qu'une quatrième réaction d'amplification comporte l'amplification d'un gène ou d'un produit de gène, ou d'une partie d'un tel gène ou produit, de *Staphylococcus* spp., *Enterococcus* spp., *Klebsiella* spp., et *Candida albicans* ;
- et qu'une cinquième réaction d'amplification comporte l'amplification des gènes *vanA* et *ctxM* ou des produits de ces gènes, ou de parties de ces gènes ou produits.

9. Procédé conforme à l'une des revendications 7 et 8, dans lequel ledit gène d'*Escherichia coli* est le gène *gadA* et/ou *gadB,* et dans lequel ladite réaction d'amplification d'acides nucléiques est une réaction PCR en temps réel dans laquelle on utilise comme amorce sens la séquence 5'-GGCTTCGAAATGGACTTTGCT-3', comme amorce anti-sens la séquence 5'-TGGGCAATACCCTGCAGTTT-3', et comme sonde la séquence 5'-CTGTTGCTGGAAGACTACAAAGCCTCCCTG-3'.

10. Procédé conforme à l'une des revendications 7 à 9, dans lequel ledit gène d'*Enterococcus faecalis* est le gène *ref12A,* et dans lequel ladite réaction d'amplification d'acides nucléiques est une réaction PCR en temps réel dans laquelle on utilise comme amorce sens la séquence 5'-ATGCGTCTCGTCACAGTA-3', comme amorce anti-sens la séquence 5'-GGTACGATGATTTCATCTGT-3', et comme sonde la séquence 5'-AGTTGCGATGTTTCACTGTGAAGCA-3'.

11. Procédé conforme à l'une des revendications 7 à 10, dans lequel ledit gène de *Streptococcus pneumoniae* est le gène *comX,* et dans lequel ladite réaction d'amplification d'acides nucléiques est une réaction PCR en temps réel dans laquelle on utilise comme amorce sens la séquence 5'-GGTCTCTGGCTAGATGATTATTATCTCTT-3', comme amorce anti-sens la séquence 5'-ATAGTAAACTCCTTAAACACAAT-GCGTAA-3', et comme sonde la séquence 5'-CGCCCTCGAAATCGT-TCATTGCTTAAGA-3'.

12. Procédé conforme à l'une des revendications 7 à 11, dans lequel :
- ledit gène d'*Aspergillus* spp. est la région ITS d'ARNr 18S-28S, et ladite réaction d'amplification d'acides nucléiques est une réaction PCR en temps réel dans laquelle on utilise comme amorce sens la séquence 5'-GCGTCATTGCTGCCCTCAAGC-3', comme amorce anti-sens la séquence 5'-ATATGCTTAAGTTCAGCGGGT-3', et comme sonde la séquence 5'-CCTCGAGCGTATGGGGC-3' ;
- et/ou ledit gène de bactéries Gram-positives est le gène d'ADNr 16S, et ladite réaction d'amplification d'acides nucléiques est une réaction PCR en temps réel dans laquelle on utilise comme amorce sens la séquence 5'-TGGAGCATGTGGTTTAATTCGA-3', comme amorce anti-sens la séquence 5'-TGCGGGACTTAACCCAACA-3', et comme sonde la séquence 5'-TGGTGCATGGTTG-3' ;
- et/ou ledit gène de bactéries Gram-négatives est le gène d'ADNr 16S, et ladite réaction d'amplification d'acides nucléiques est une réaction PCR en temps réel dans laquelle on utilise comme amorce sens la séquence 5'-TGGAGCATGTGGTTTAATTCGA-3', comme amorce anti-sens la séquence 5'-TGCGGGACTTAACCCAACA-3', et comme sonde la séquence 5'-TGCTGCATGGCTGT-3' ;
- et/ou ledit gène de *Candida* spp. est la région ITS d'ARNr 18S-28S, et ladite réaction d'amplification d'acides nucléiques est une réaction PCR en temps réel dans laquelle on utilise comme amorce sens la séquence 5'-CATGCCTGTTTGAGCGTCRTTT-3', comme amorce anti-sens la séquence 5'-ATATGCTTAAGTTCAGCGGGT-3', et comme sonde la séquence 5'-TCGTATTGCTCAACACCAAACCC-3' ;
- et/ou ledit gène de *Candida glabrata* est la région ITS d'ARNr 18S-28S, et ladite réaction d'amplification d'acides nucléiques est une réaction PCR en temps réel dans laquelle on utilise comme amorce sens la séquence 5'-CATGCCTGTTTGAGCGTCRTTT-3', comme amorce anti-sens la séquence 5'-ATATGCTTAAGTTCAGCGGGT-3', et comme sonde la séquence 5'-ATCAGTATGTGGGACACGAGCG-3' ;
- et/ou ledit gène est le gène *mecA* ou une partie de celui-ci, et ladite réaction d'amplification d'acides nucléiques est une réaction PCR en temps réel dans laquelle on utilise comme amorce sens la séquence 5'-GATCGCAACGTTCAATTTAATTTT-3', comme amorce anti-sens la séquence 5'-GCTTTGGTCTTTCTGCATTCCT-3', et comme sonde la séquence 5'-AATGACGCTATGATCCCAATCTAACTTCCACAT-3' ;
- et/ou ledit gène de *Candida brusei* est la région ITS d'ARNr 18S-28S, et ladite réaction d'amplification d'acides nucléiques est une réaction PCR en temps réel dans laquelle on utilise comme amorce sens la séquence 5'-CATGCCTGTTTGAGCGTCRTTT-3', comme amorce anti-sens la séquence 5'-ATATGCTTAAGTTCAGCGGGT-3', et comme sonde la séquence 5'-ACGACGTGTAAAGAGCGTCGG-3' ;
- et/ou ledit gène d'*Enterococcus* spp. est le gène d'ADNr 23S, et ladite réaction d'amplification d'acides nucléiques est une réaction PCR en temps réel dans laquelle on utilise comme amorce sens la séquence 5'-TGCGGGGATGAGGTGTG-3', comme amorce anti-sens la séquence 5'-CAAACAGTGCTCTACCTCCATCAT-3', et comme sonde la séquence 5'-TAGCCCTAAAGCTATTTCGGAGAGAACCA-3' ;
- et/ou ledit gène de *Candida albicans* est la région ITS d'ARNr 18S-28S, et ladite réaction d'amplification d'acides nucléiques est une réaction PCR en temps réel dans laquelle on utilise comme amorce sens la séquence 5'-CATGCCTGTTTGAGCGTCRTTT-3', comme amorce anti-sens la séquence 5'-ATATGCTTAAGTTCAGCGGGT-3', et comme sonde la séquence 5'-TAAGGCGGGATCGCTTTGACA-3' ;
- et/ou ledit gène est le gène *vanA* ou une partie de celui-ci, et ladite réaction d'amplification d'acides nucléiques est une réaction PCR en temps réel dans laquelle on utilise comme amorce sens la séquence 5'-CGGTTTCACGTCATACAGTCGTT-3', comme amorce anti-sens la séquence 5'-CAGTTCGGGAAGTGCAATACC-3', et comme sonde la séquence 5'-TCCCCGTATGATGGCCGCTGC-3' ;
- et/ou ledit gène est le gène *ctxM-1* ou une partie de celui-ci, et ladite réaction d'amplification d'acides nucléiques est une réaction PCR en temps réel dans laquelle on utilise comme amorce sens la séquence 5'-ATGTGCAGYACCAGTAARGTKATGGC-3', comme amorce anti-sens la séquence 5'-ATCACKCGGRTCGCCNGGRAT-3', et comme sonde la séquence 5'-CCCGACAGCTGGGAGACGAAACGT-3' ;
- et/ou ledit gène est le gène *ctxM-9* ou une partie de celui-ci, et ladite réaction d'amplification d'acides nucléiques est une réaction PCR en temps réel dans laquelle on utilise comme amorce sens la séquence 5'-ATGTGCAGYACCAGTAARGTKATGGC-3', comme amorce anti-sens la séquence 5'-ATCACKCGGRTCGCCNGGRAT-3', et comme sonde la séquence 5'-CTGGATCGCACTGAACCTACGCTGA-3'.

13. Jeu de parties adaptées pour exécuter un procédé conforme à l'une des revendications 1 à 12, lequel jeu comprend au moins une amorce sens ou anti-sens ou sonde définie dans l'une des revendications 2 et 9 à 12, et dans lequel jeu, de préférence, au moins l'une de ces amorces sens ou anti-sens ou sondes au nombre d'au moins une porte un marqueur détectable, de préférence un marqueur fluorescent.

14. Procédé pour surveiller une septicémie ou déterminer l'efficacité d'un traitement antiseptique, lequel procédé comporte l'exécution d'un procédé conforme à l'une des revendications 1 à 13 sur au moins deux échantillons, lesquels échantillons sont obtenus à différents moments au cours de la septicémie ou à différents moments au cours du traitement antiseptique.

15. Antibiotique spécifique pour utilisation dans un procédé de traitement d'un sujet souffrant ou suspecté de souffrir d'une septicémie, lequel procédé comporte le fait de traiter le sujet avec ledit antibiotique spécifique, étant entendu qu'on a détecté dans un échantillon dudit sujet, par un procédé conforme à l'une des revendications 1 à 13, l'un ou plusieurs des micro-organismes suivants : *Pseudomonas aeruginosa, Klebsiella species, Escherichia coli, Acinetobacter baumannii, Enterococcus faecalis, Enterococcus faecium, Staphylococcus species, Staphylococcus aureus, Streptococcus pneumoniae, Streptococcus agalactiae, Serratia marcescens, Candida albicans, Candida glabrata, Candida krusei, pan-Aspergillus, pan-Candida,* bactéries Gram-positives, bactéries Gram-négatives, *MecA, VanA,* et/ou *CTXM.*
